# EUROPEAN PATENT SPECIFICATION

(11) **EP 4 257 585 B1**
(45) Date of publication and mention of the grant of the patent: **07.10.2026**
(21) Application number: 21900661.6
(22) Date of filing: 02.12.2021
(51) Int. Cl.: C07D 213/16, C07D 239/26, C07D 401/14, C07D 403/10, C07D 405/04, C07D 405/10, C07D 409/10, C07D 409/14, C07D 413/10, C07D 417/10, C07D 471/04, C07D 519/00, C09K 11/06, H05B 33/22, H05B 33/10, H05B 33/00, H01J 61/42

(54) **HETEROCYCLIC COMPOUND, ORGANIC ELECTROLUMINESCENT DEVICE, AND ELECTRONIC APPARATUS**
HETEROCYCLISCHE VERBINDUNG, ORGANISCHES ELEKTROLUMINESZENZELEMENT UND ELEKTRONISCHE VORRICHTUNG
COMPOSÉ HÉTÉROCYCLIQUE, ÉLÉMENT ÉLECTROLUMINESCENT ORGANIQUE, ET APPAREIL ÉLECTRONIQUE

(30) Priority: 02.12.2020 JP 2020199960
(43) Date of publication of application: 11.10.2023
(73) Proprietor: Hodogaya Chemical Co., Ltd., Tokyo, 105-0021 (JP)
(72) Inventor: YAMAMOTO, Takeshi, Tokyo 105-0021 (JP); KASE, Kouki, Tokyo 105-0021 (JP); HIRAYAMA, Yuta, Tokyo 105-0021 (JP); PARK, Kyung-Hwa, Tokyo 105-0021 (JP); HAYASHI, Shuichi, Tokyo 105-0021 (JP)
(74) Representative: Müller-Boré & Partner Patentanwälte PartG mbB
(86) International application number: PCT/JP2021/044199
(87) International publication number: WO 2022/118910

(56) References cited:
- EP-A2- 2 168 576
- EP-B1- 2 331 655
- CN-A- 107 417 677
- CN-A- 109 705 148
- CN-A- 110 283 143
- CN-A- 111 253 319
- JP-A- 2019 518 716
- KR-B1- 102 052 332
- KR-B1- 102 080 737
- US-A1- 2020 203 626
- US-A1- 2020 207 767
- US-B2- 9 966 541

## Description

### Technical Field

The present invention relates to a heterocyclic compound, suitable for a preferred self-luminous electron device for various display devices, particularly an organic electroluminescent device (hereinafter referred to as organic EL device), or an electronic apparatus, and an organic EL device or an electronic apparatus using the heterocyclic compound.

### Background Art

In 1987, C.W. Tang and colleagues at Eastman Kodak developed a laminated structure device using materials assigned with different roles, realizing practical applications of an organic EL device with organic materials. These researchers laminated an electron-transporting phosphor and a hole-transporting organic substance and injected both charges into a phosphor layer to cause emission to obtain a high luminance of 1,000 cd/m² or more at a voltage of 10 V or less (see PTLs 1 and 2).

To date, many improvements have been made for the practical application of organic EL devices. Various roles of the laminated structure have been further subdivided to provide a light emitting device that includes an anode, a hole injection layer, a hole transport layer, a light emitting layer, an electron transport layer, an electron injection layer, and a cathode sequentially on a substrate. In this light emitting device, high efficiency and durability have come to be achieved by providing thereto a bottom emission structure that emits light from the bottom (see, for example, NPL 1).

Recently, a light emitting device with a top emission structure in which a metal having a high work function is used for an anode and light is emitted from the top is coming into use. In a light emitting device with a bottom-emission structure where light is taken out from the bottom having a pixel circuit, the area of the light emitting portion is limited, whereas in a light emitting device with a top-emission structure has the advantage that the light is taken out from the top and is not blocked by the pixel circuit, thus allowing a larger emission area. In the light emitting device with a top emission structure, translucent electrodes made of LiF/Al/Ag (see, for example, NPL 2), Ca/Mg (see, for example, NPL 3), LiF/MgAg, or the like are used for a cathode.

In such light emitting device, when light emitted by a light emitting layer enters another layer at a certain angle or more, the light is totally reflected at an interface between the light emitting layer and the other layer. Thus, light that can be used has been limited to only a part of the emitted light. Recently, a light emitting device has been proposed in which a "capping layer" with a high refractive index is provided on the outside of a translucent electrode with a low refractive index, in order to improve the light extraction efficiency (see, for example, NPLs 2 and 3).

Regarding the effect of the capping layer in a light emitting device with a top emission structure, while a light emitting device using Ir(ppy)₃ as a light emitting material has a current efficiency of 38 cd/A in the case of not having a capping layer, the light emitting device has a current efficiency of 64 cd/A, in the case of using a ZnSe film with a thickness of 60 nm as a capping layer, which indicates that the efficiency is improved about 1.7 times. Furthermore, it is indicated that the maximum point of transmittance of the translucent electrode and capping layer does not necessarily coincide with the maximum point of efficiency, and that the maximum point of light extraction efficiency is determined by interference effects (see, for example, NPL 3).

In the past, it has been proposed to use a metal mask with a high degree of definition to form the capping layer, but there is a problem that the metal mask is distorted by heat when used under high temperature conditions, resulting in a decrease in alignment accuracy. Therefore, ZnSe has a high melting point of 1100°C or higher (see, for example, NPL 3), and a high definition metal mask cannot be used to deposit ZnSe at precise positions, which may affect the light emitting devices themselves. Furthermore, even deposition by the sputtering method affects the light emitting devices, inorganic materials are not suitable for use as components of the capping layer.

In addition, when tris(8-hydroxyquinoline)aluminum (Alq₃) is used as a capping layer to adjust the refractive index (see, for example, NPL 2), Alq₃ is known as an organic EL material commonly used as a green emitting material or electron transport material, it has a weak absorption around 450 nm, which is used for blue emitting materials, and has the problems of reducing the color purity and light extraction efficiency of blue emitting devices. PTL 3 describes optical retardation films with biaxial properties for liquid crystal displays. PTL 4 describes a pharmaceutical composition for enhancing the expression of apolipoprotein Al. PTL 5 describes an organic electroluminescent (EL) device such as an organic light emitting device (hereinafter abbreviated as an OLED) and materials capable of being used in such an OLED. PTL 6 describes a novel organic compound containing anthryl and phenanthrolinyl group, and a display panel containing the organic compound and a display apparatus including the display panel. PTL 7 describes a carbazole pyridine organic electroluminescent material and an organic electroluminescent device thereof, belonging to the technical field of organic electroluminescent device display. PTL 8 describes a phenanthroline compound and an organic light-emitting device comprising the same. PTL 9 describes an organic light-emitting compound. PTL 10 describes a nitrogen heterocyclic compound and a display panel and a display device comprising the nitrogen heterocyclic compound. PTL 11 describes an azapyrene compound, a display panel including the azapyrene compound, and a display apparatus. PTL 12 describes an aromatic amine compound and an organic light-emitting device containing the compound. PTL 13 describes an aromatic ring compound and a display panel and a display device comprising the aromatic ring compound. PTL 14 describes organic compounds having triazine and benzimidazole cores and organic electroluminescent devices using said compounds.

In order to improve the device characteristics of an organic EL device, and to significantly improve the light extraction efficiency, materials with high refractive index and low extinction coefficient, and excellent thin film stability and durability are required as capping layer materials.

### Citation List

### Patent literatures

PTL 1: JP H08-048656 A
PTL 2: JP 3194657 B
PTL 3: EP 2 331 655 B
PTL 4: EP 2 168 576 A2
PTL 5: US 9 966 541 B2
PTL 6: US 2020/203626 A1
PTL 7: CN 107 417 677 A
PTL 8: KR 102 052 332 B1
PTL 9: KR 102 080 737 B1
PTL 10: CN 111 253 319 A
PTL 11: US 2020/207767 A1
PTL 12: CN 110 283 143 A
PTL 13: CN 109 705 148 A
PTL 14: JP 2019 518716 A

### Non-Patent Literatures

NPL 1: Proceedings of the 9th Meeting of the Japan Society of Applied Physics, pp. 55 to 61 (2001)
NPL 2: Appl.Phys.Let.,78,544(2001)
NPL 3: Appl.Phys.Let.,82,466(2003)

### Summary of the Invention

### Technical Problem

An object of the present invention is to provide a compound having high refractive index at a wavelength of light transmitted through a capping layer (an organic thin film) of an organic EL device in range of 450nm and 750 nm, and no absorption in around 450 nm. Furthermore, it is to provide an organic EL device, or an electronic apparatus or electron device improving light extraction efficiency by using the compound.

Physical properties of the compound constituting the capping layer (the organic thin film) suitable for the present invention include (1) a high refractive index, (2) vapor deposition ability, (3) a stable thin film state, and (4) a high glass transition temperature. In addition, physical properties of the device suitable for the present invention include that (1) the light extraction efficiency is high, (2) the color purity does not decrease, (3) it transmits light without change over time, and (4) the lifetime is long.

### Solution to Problem

For achieving the object, the present inventors focused on the fact that heterocyclic compounds have excellent thin film stability and durability, and is able to improve the refractive index by adjusting the molecular structures, and planed molecule, and fabricated organic EL devices using the heterocyclic compounds as the materials constituting the capping layer, and thoroughly evaluated the properties of the devices, as a result of which the present invention was accomplished.

Specifically, according to the present invention, the heterocyclic compounds and organic EL devices as defined in the appended set of claims are provided.

1) A heterocyclic compound represented by the following general formula (1).

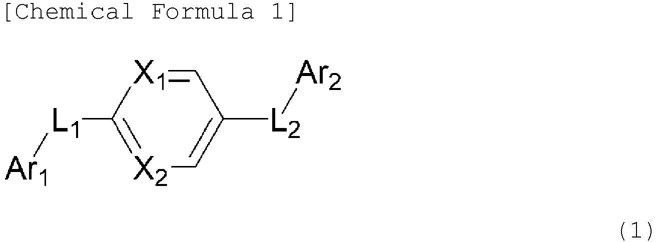

In the formula, X₁ and X₂ may be the same or different, and represent a nitrogen atom, or a CH group, at least one of X₁ and X₂ is a nitrogen atom. L₁ and L₂ may be the same or different, and represent a single bond, or a divalent group that results from the removal of two hydrogen atoms from substituted or unsubstituted benzene, a divalent group that results from the removal of two hydrogen atoms from substituted or unsubstituted biphenyl, or a divalent group that results from the removal of two hydrogen atoms from substituted or unsubstituted naphthalene. Ar₁ and Ar₂ may be the same or different, and represent a substituted or unsubstituted aromatic hydrocarbon group selected from the group consisting of a naphthyl group, a phenanthrenyl group, and a spirobifluorenyl group, or a substituted or unsubstituted aromatic heterocyclic group selected from the group consisting of a quinolyl group, a benzofuranyl group, a benzothienyl group, a carbazolyl group, a dibenzothienyl group, a phenanthrolinyl group, a benzoxazolyl group, and a benzothiazolyl group.

2) The heterocyclic compound according to item 1), wherein the heterocyclic compound is represented by the following general formula (1-a).

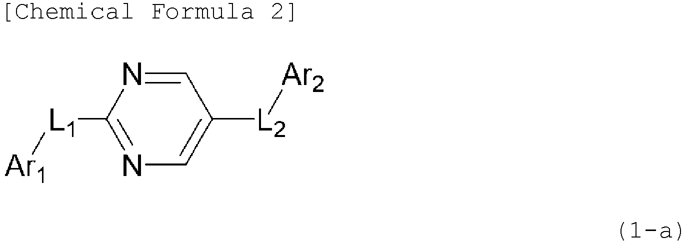

In the formula, L₁, L₂, Ar₁ and Ar₂ are as defined in the general formula (1).

3) The heterocyclic compound according to item 1), wherein the heterocyclic compound is represented by the following general formula (1-b).

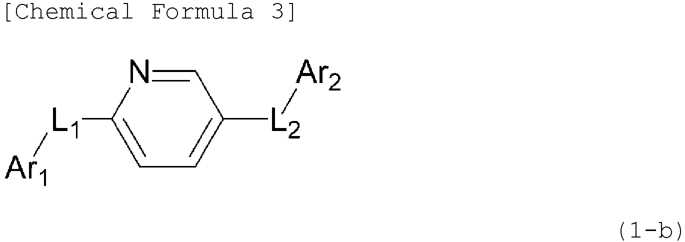

In the formula, L₁, L₂, Ar₁ and Ar₂ are as defined in the general formula (1).

4) The heterocyclic compound according to any one of items 1) to 3), wherein in the general formula (1), in the general formula (1-a), or in the general formula (1-b), at least one of L₁ and L₂ is represented by the following the general formula (2).

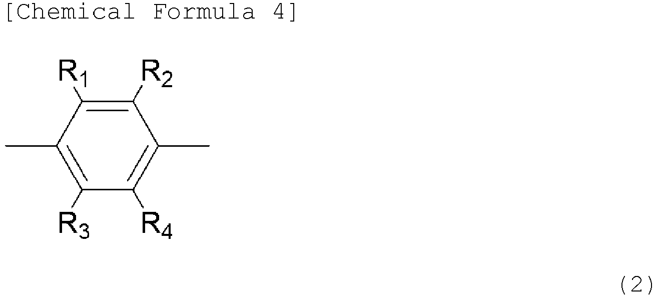

In the formula, R₁ to R₄ represent a hydrogen atom, a cyano group, a substituted or unsubstituted alkyl group of 1 to 6 carbon atoms, a substituted or unsubstituted alkyloxy group of 1 to 6 carbon atoms, or a substituted or unsubstituted aromatic hydrocarbon group of 6 to 12 ring-forming carbon atoms.

5) An organic thin film comprising the heterocyclic compound according to any one of items 1) to 4), wherein the organic thin film has a refractive index of 1.70 or more at a wavelength in range of 450 nm and 750 nm.

6) An organic EL device comprising at least an anode electrode, a hole transport layer, a light emitting layer, an electron transport layer, a cathode electrode, and a capping layer in this order, wherein the capping layer is the organic thin film according to item 5).

7) An electronic apparatus or an electronic device comprising a pair of electrodes and at least one organic layer sandwiched therebetween, wherein the heterocyclic compound according to any one of items 1) to ) is used for the organic layer as a constituent material thereof.

In the present invention, specific examples of the "substituent" in the "substituted or unsubstituted" include a cyano group, a nitro group, a halogen atom; an alkyl group of 1 to 6 carbon atoms, such as a methyl group, an ethyl group, an n-propyl group, an isopropyl group, an n-butyl group, an isobutyl group, a tert-butyl group, an n-pentyl group, an isopentyl group, a neopentyl group, and an n-hexyl group; an alkyloxy group of 1 to 6 carbon atoms, such as a methyloxy group, an ethyloxy group, an n-propyloxy group, an isopropyloxy group, an n-butyloxy group, an isobutyloxy group, a tert-butyloxy group, an n-pentyloxy group, an isopentyloxy group, a neopentyloxy group, and an n-hexyloxy group; an aromatic hydrocarbon group, such as a phenyl group, a biphenyl group, a terphenyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group; and an aromatic heterocyclic group, such as a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a quinoxalinyl group, a benzoimidazolyl group, a pyrazolyl group, a dibenzofuranyl group, a dibenzothienyl group, a naphthyridinyl group, a phenanthrolinyl group, an acridinyl group, a carbolinyl group, a benzoxazolyl group, a benzothiazolyl group, and a phenoxazinyl group. These substituents may be further substituted with the exemplified substituents above.

In the present invention, a cyano group, a nitro group, a halogen atom, alkyl group of 1 to 3 carbon atoms, alkyloxy group of 1 to 3 carbon atoms, a phenyl group, a naphthyl group, or a quinolyl group is preferably used among the exemplified "substituents" above.

Here, a divalent group that results from the removal of two hydrogen atoms from benzene (i.e., a phenylene group), a divalent group that results from the removal of two hydrogen atoms from biphenyl, or a divalent group that results from the removal of two hydrogen atoms from naphthalene is preferably a divalent group that results from the removal of two hydrogen atoms from benzene (i.e., a phenylene group), or a divalent group that results from the removal of two hydrogen atoms from naphthalene.

The divalent group that results from the removal of two hydrogen atoms from benzene (i.e., a phenylene group) is preferably a divalent group that results from the removal of two hydrogen atoms at the 1,4-positions from benzene (i.e., a 1,4-phenylene group), or a divalent group that results from the removal of two hydrogen atoms at the 1,3-positions from benzene (i.e., a 1,3-phenylene group).

The divalent group that results from the removal of two hydrogen atoms from biphenyl is preferably a divalent group that results from the removal of two hydrogen atoms at the 4,4'-positions from biphenyl.

The divalent group that results from the removal of two hydrogen atoms from naphthalene is preferably a divalent group that results from the removal of two hydrogen atoms at the 1,4-positions from naphthalene.

The "aromatic hydrocarbon group" in the "substituted or unsubstituted aromatic hydrocarbon group" represented by Ar₁, Ar₂ in the general formula (1) include a naphthyl group, a phenanthrenyl group, and a spirobifluorenyl group.

The "aromatic heterocyclic group" in the "substituted or unsubstituted aromatic heterocyclic group" represented by Ar₁, Ar₂ in the general formula (1) include a quinolyl group a benzofuranyl group, a benzothienyl group, a carbazolyl group, a dibenzothienyl group, a phenanthrolinyl group, a benzoxazolyl group, and a benzothiazolyl group.

Specific examples of the "alkyl group of 1 to 6 carbon atoms" in the "substituted or unsubstituted alkyl group of 1 to 6 carbon atoms" represented by R₁ to R₄ in the general formula (2) include a methyl group, an ethyl group, an n-propyl group, an isopropyl group, an n-butyl group, an isobutyl group, a tert-butyl group, an n-pentyl group, an isopentyl group, a neopentyl group, and an n-hexyl group.

Specific examples of the "alkyloxy group of 1 to 6 carbon atoms" in the "substituted or unsubstituted alkyloxy group of 1 to 6 carbon atoms" represented by R₁ to R₄ in the general formula (2) include a methyloxy group, an ethyloxy group, an n-propyloxy group, an isopropyloxy group, an n-butyloxy group, an isobutyloxy group, a tert-butyloxy group, an n-pentyloxy group, an isopentyloxy group, a neopentyloxy group, and an n-hexyloxy group.

Specific examples of the "aromatic hydrocarbon group of 6 to 12 ring-forming carbon atoms" in the "substituted or unsubstituted aromatic hydrocarbon group of 6 to 12 ring-forming carbon atoms" represented by R₁ to R₄ in the general formula (2) include a phenyl group, a biphenyl group, and a naphthyl group.

The heterocyclic compound represented by the general formula (1) of the present invention has preferably a refractive index of 1.70 or more at a wavelength in range of 450 nm to 750 nm, more preferably, 1.80 or more, further preferably 1.85 or more.

The heterocyclic compound represented by the general formula (1) of the present invention is preferably the heterocyclic compound represented by (1-a) or (1-b).

In the general formula (1), the general formula(1-a), or the general formula(1-b), at least one of L₁, L₂ is preferably a divalent group represented by the general formula (2).

In the general formula (2), R₁ to R₄ are preferably a hydrogen atom, more preferably, all of R₁ to R₄ are hydrogen atoms.

In the organic EL device of the present invention, the thickness of the capping layer is preferably in a range of 30 nm to 120 nm, and more preferably in a range of 40 nm to 80 nm.

In the organic EL device of the present invention, the capping layer may be produced as a laminated layer or a mixed layer of two or more kinds of different constitutional materials.

In the organic EL device of the present invention, the refractive index of the capping layer to light transmitted through the capping layer having a wavelength in a range of 450 nm to 750 nm is preferably 1.70 or more, more preferably 1.80 or more, and further preferably 1.85 or more.

### Advantageous Effects of Invention

The heterocyclic compound of the present invention is used for a capping layer with a higher refractive index than that of the transparent or translucent electrode of the organic EL device, which is provided outside the transparent or translucent electrode, making it possible to obtain an organic EL device that can significantly improve the light extraction efficiency.

In addition, the heterocyclic compound of the present invention can be used not only in an organic EL device, but also in the field of electronic apparatus such as an electrophotographic photoreceptor, an image sensor, a photoelectric conversion device, and a solar cell.

### Brief Description of Drawings

FIG. 1 is a figure showing the structural formulas of compounds (1-1) to (1-16) as a heterocyclic compound represented by the general formula (1) of the present invention.
FIG. 2 is a figure showing the structural formulas of compounds (1-17) to (1-34) as the heterocyclic compound represented by the general formula (1) of the present invention.
FIG. 3 is a figure showing the structural formulas of compounds (1-35) to (1-50) as the heterocyclic compound represented by the general formula (1) of the present invention.
FIG. 4 is a figure showing the structural formulas of compounds (1-51) to (1-68) as the heterocyclic compound represented by the general formula (1) of the present invention.
FIG. 5 is a figure showing the structural formulas of compounds (1-69) to (1-82) as the heterocyclic compound represented by the general formula (1) of the present invention.
FIG. 6 is a figure showing the structural formulas of compounds (1-83) to (1-96) as the heterocyclic compound represented by the general formula (1) of the present invention.
FIG. 7 is a figure showing the structural formulas of compounds (1-97) to (1-110) as the heterocyclic compound represented by the general formula (1) of the present invention.
FIG. 8 is a figure showing the structural formulas of compounds (1-111) to (1-124) as the heterocyclic compound represented by the general formula (1) of the present invention.
FIG. 9 is a figure showing the structural formulas of compounds (1-125) to (1-138) as the heterocyclic compound represented by the general formula (1) of the present invention.
FIG. 10 is a figure showing the structural formulas of compounds (1-139) to (1-152) as the heterocyclic compound represented by the general formula (1) of the present invention.
FIG. 11 is a figure showing the structural formulas of compounds (1-153) to (1-155) as the heterocyclic compound represented by the general formula (1) of the present invention.
FIG. 12 is a diagram illustrating the configuration of an organic EL devices of Examples 21 to 38 and Comparative Examples 1 to 2.

### Description of Embodiments

The heterocyclic compounds represented by the general formula (1) of the present invention are novel compounds, but these compounds per se can be synthesized according to a known method.

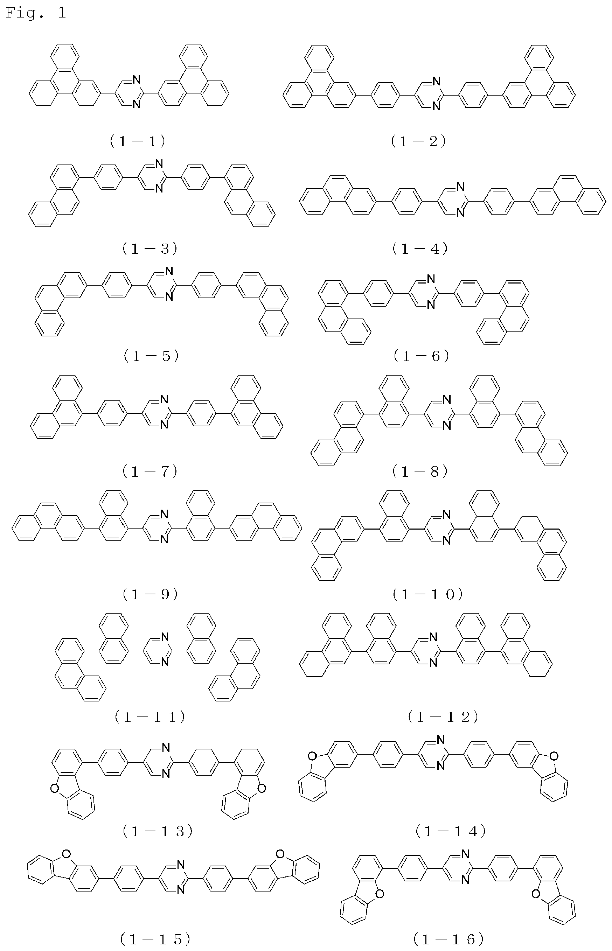

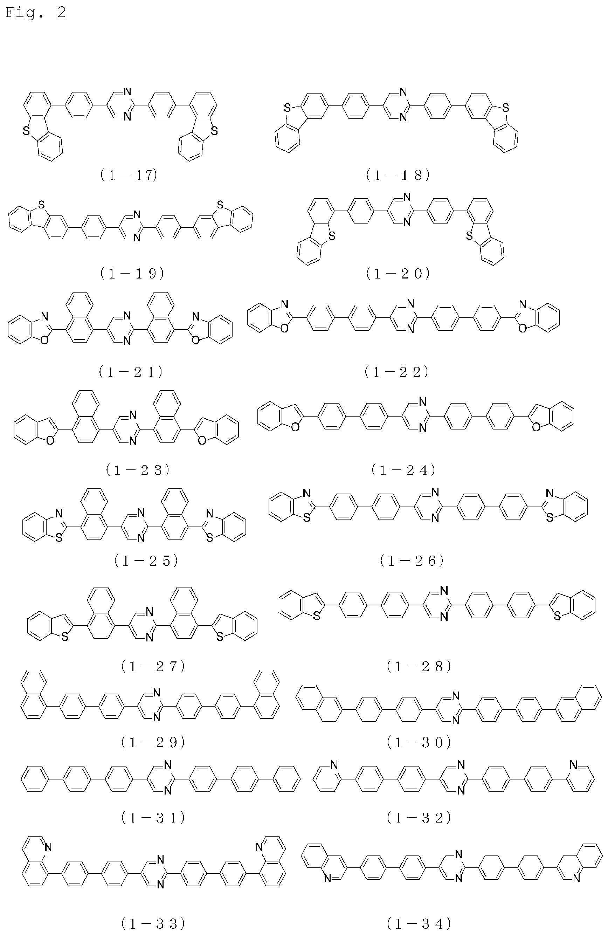

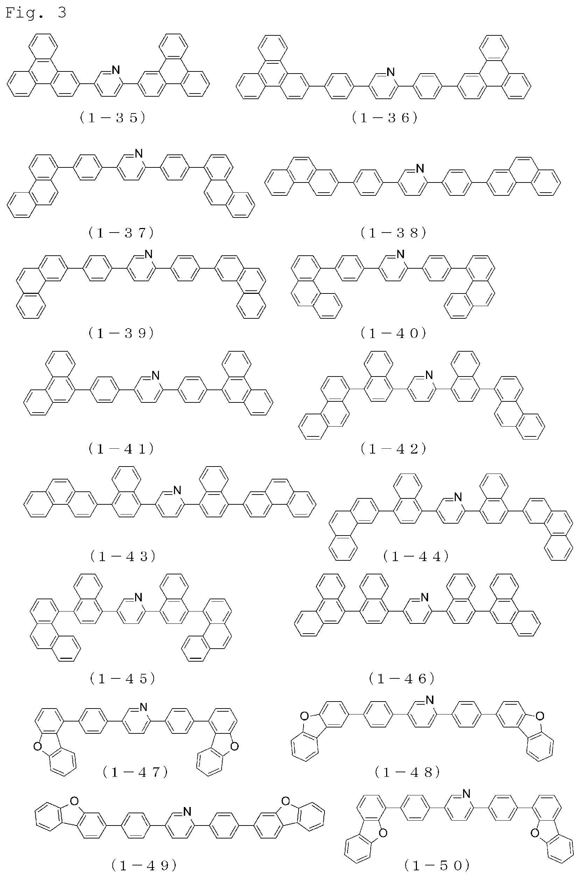

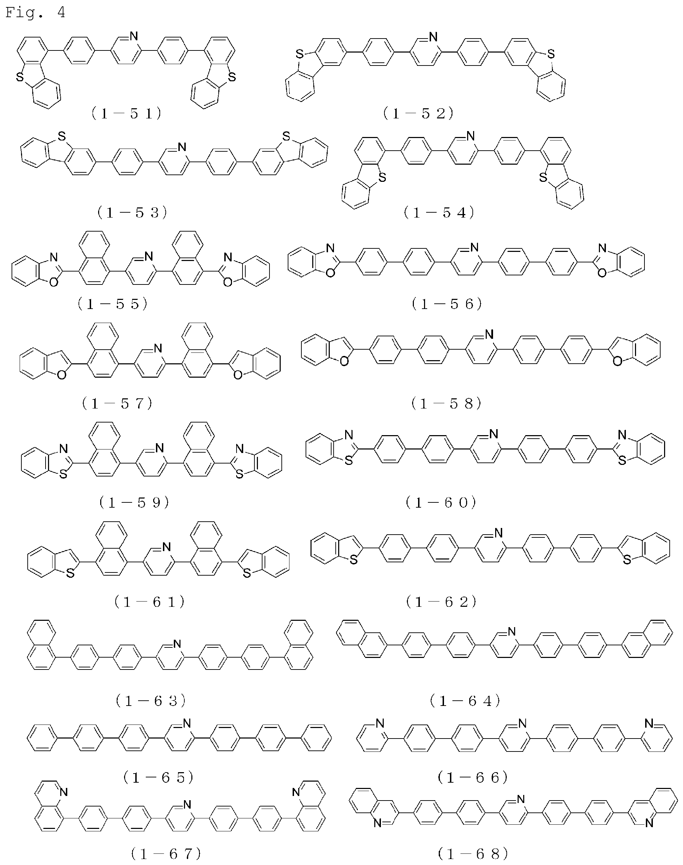

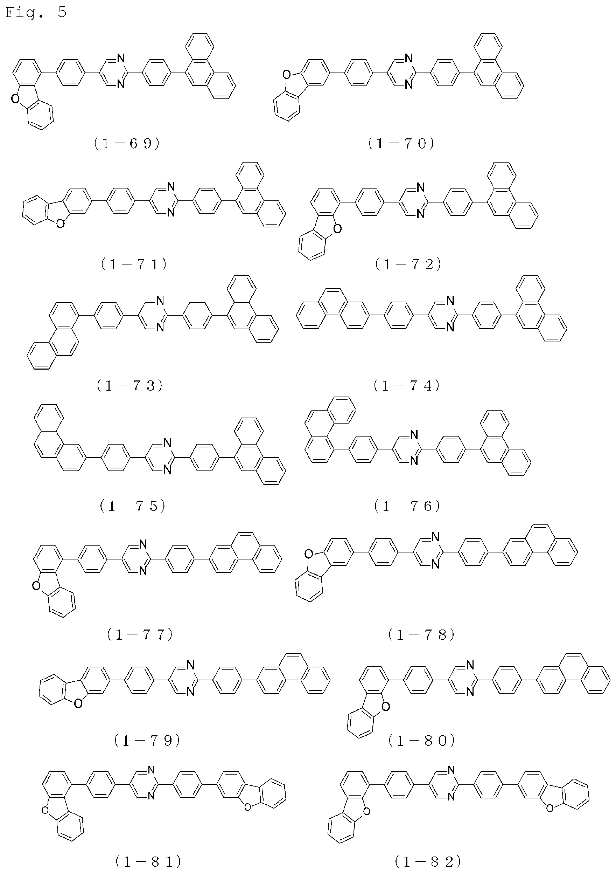

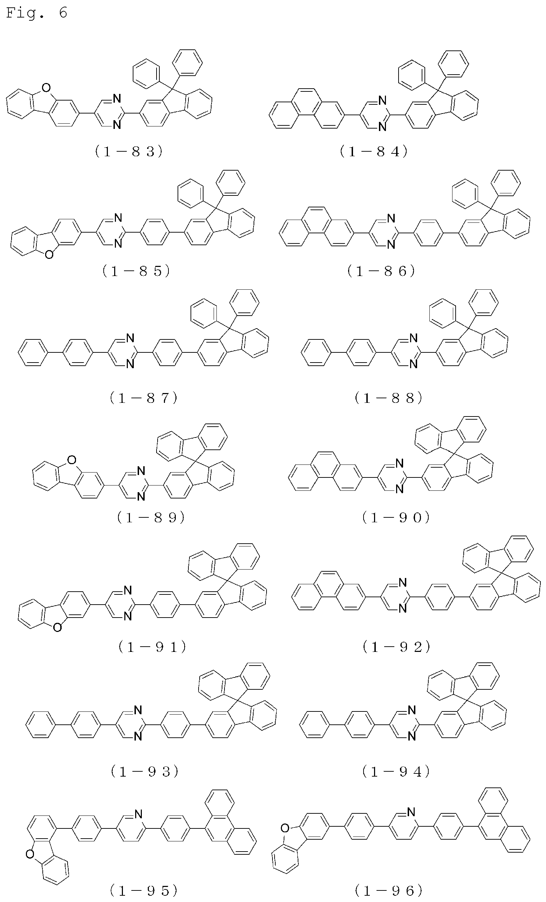

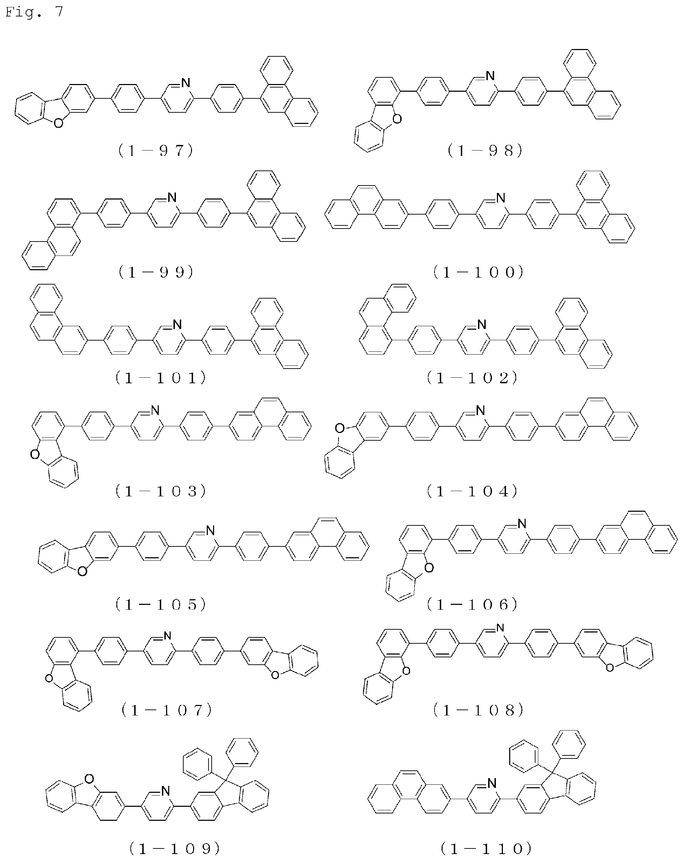

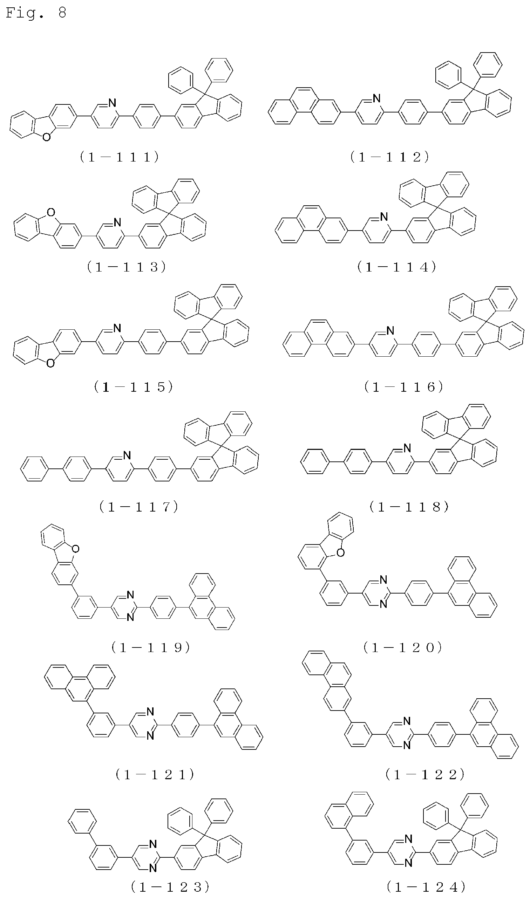

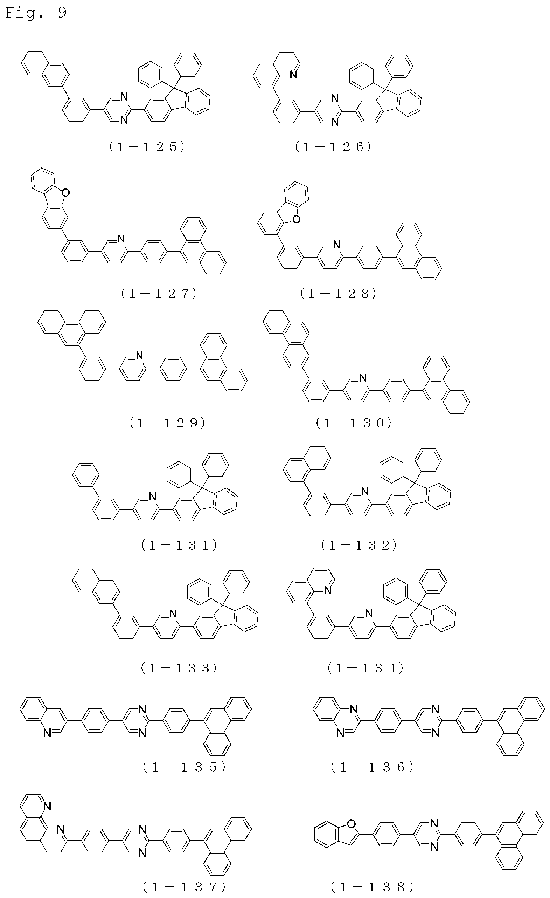

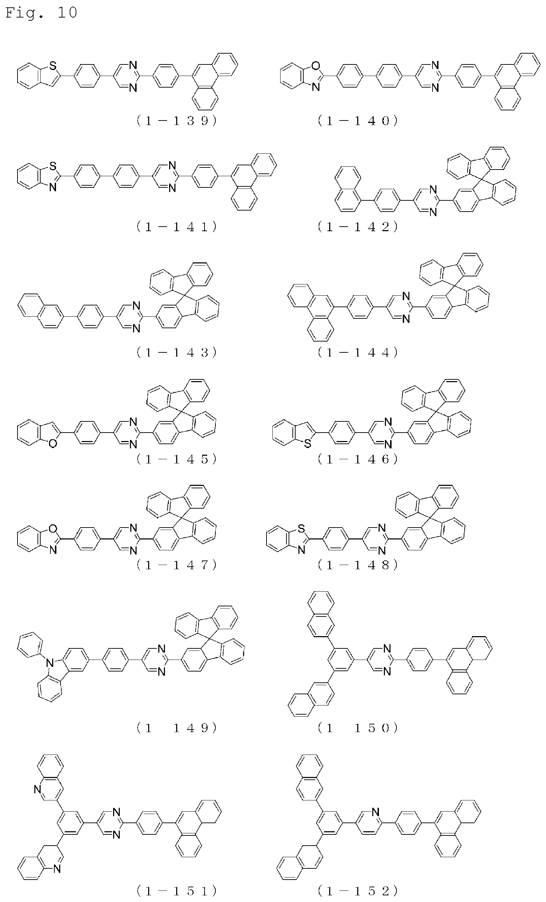

The specific examples of the heterocyclic compound represented by the general formula (1) of the present invention are shown in Figs. 1 to 11. The present invention, however, is not restricted to these compounds.

A method for producing the heterocyclic compound represented by the general formula (1) of the present invention is not particularly limited, but the purification of the compound can be performed by a known method used for purifying an organic compound such as purification using a column chromatograph, adsorption purification using silica gel, activated carbon, activated clay, or the like, recrystallization or crystallization using a solvent, and finally, purification by a sublimation purification method or the like was performed. The identification of the compound can be performed by an NMR analysis, or the like. It is preferred to measure a melting point, a glass transition point (Tg), and a refractive index as physical property values of the compound.

The melting point and the glass transition point (Tg) can be measured by, for example, a high-sensitive differential scanning calorimeter (DSC3100SA manufactured by Bruker AXS) using a powder.

The refractive index was measured using a spectrometer (F10-RT-UV manufactured by Filmetrics) by preparing a thin film of 80 nm on a silicon substrate.

The organic EL device of the present invention, for example, in the case of a top emission light emitting device, may have a structure including an anode, a hole transport layer, a light emitting layer, an electron transport layer, a cathode, and a capping layer successively formed on a glass substrate, optionally with a hole injection layer between the anode and the hole transport layer, an electron blocking layer between the hole transport layer and the light emitting layer, a hole blocking layer between the light emitting layer and the electron transport layer, and an electron injection layer between the electron transport layer and the cathode. Some of the organic layers in the multilayer structure may be omitted or may serve more than one function. For example, a single organic layer may serve as a hole injection layer and a hole transport layer, a hole transport layer and an electron blocking layer, a hole blocking layer and an electron transport layer, or an electron transport layer and an electron injection layer, and so on. Further, any of the layers may be configured to laminate two or more organic layers having the same function, and the hole transport layer may have a two-layer laminated structure, the light emitting layer may have a two-layer laminated structure, the electron transport layer may have a two-layer laminated structure, the capping layer may have a two-layer laminated structure, and so on.

The total film thickness of the respective layers of the organic EL device is preferably about 200 nm to 750 nm, and more preferably about 350 nm to 600 nm. Further, the film thickness of the capping layer is, for example, preferably 30 nm to 120 nm, and more preferably 40 nm to 80 nm. In this case, good light extraction efficiency is obtained. The film thickness of the capping layer can be appropriately changed according to the type of light emitting material used in the light emitting device, the thickness of each layer of the organic EL device other than the capping layer, or the like.

An electrode material with a large work function, such as ITO and gold, may be used as the anode of the organic EL device of the present invention.

The hole injection layer of the organic EL device of the present invention is preferably an arylamine compound having a structure in which two or more triphenylamine structures are joined within a molecule via a single bond or a divalent group that does not contain a heteroatom, for example, an arylamine compound having a structure in which two triphenylamine structures are joined within a molecule via a single bond or a divalent group that does not contain a heteroatom, such as a benzidine derivative, a starburst-type triphenylamine derivative, and various triphenylamine tetramers. A porphyrin compound, such as copper phthalocyanine; an accepting heterocyclic compound, such as hexacyanoazatriphenylene; and a coating-type polymer material may also be used. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

As the hole transport layer of the organic EL device of the present invention, it is preferred to use a benzidine derivative such as N,N'-diphenyl-N,N'-di(m-tolyl)benzidine (TPD), N,N'-diphenyl-N,N'-di(α-naphthyl)benzidine (NPD), or N,N,N',N'-tetrabiphenylylbenzidine, 1,1-bis[4-(di-4-tolylamino)phenyl]cyclohexane (TAPC), particularly, an arylamine compound having a structure in which two triphenylamine structures are joined in a molecule via a single bond or a divalent group that does not contain a heteroatom, for example, N,N,N',N'-tetrabiphenylylbenzidine or the like. In addition, it is preferred to use an arylamine compound having only one triphenylamine structure in a molecule, an arylamine compound having a structure in which three or more triphenylamine structures are joined in a molecule via a single bond or a divalent group that does not contain a heteroatom, for example, various triphenylamine trimers and tetramers, or the like. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. In addition, a coating-type polymer material such as poly (3, 4-ethylenedioxythiophene) (PEDOT)/poly(styrene sulfonate) (PSS) can be used as a hole injection/transport layer. These materials may be formed into a thin film by using a vapor deposition method or other known methods such as a spin coating method and an inkjet method.

Further, in the hole injection layer or the hole transport layer, a material obtained by further p-doping a material which is commonly used for the layer with trisbromophenylamine hexachloroantimony, a radialene derivative, or the like is preferred. In addition, a polymer compound having a structure of a benzidine derivative such as TPD in its partial structure, or the like can be used.

The electron blocking layer of the organic EL device of the present invention may be a compound having an electron blocking effect, including, for example, a carbazole derivative, such as 4,4',4"-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-yl-phenyl)adamantane (Ad-Cz); and a compound having a triphenylsilyl group and a triarylamine structure, as represented by 9-[4-(carbazol-9-yl)phenyl]-9-[4-(triphenylsilyl)phenyl]-9H-fluorene. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

The light emitting layer of the organic EL device of the present invention may be metal complexes of a quinolinol derivative, such as Alq₃, as well as various metal complexes, an anthracene derivative, a bis-styrylbenzene derivative, a pyrene derivative, an oxazole derivative, a polyparaphenylene vinylene derivative, or the like. Further, the light emitting layer may be made of a host material and a dopant material. An anthracene derivative is preferably used as the host material, but in addition to the light emitting materials above, a heterocyclic compound having an indole ring as a partial structure of the fused ring, a heterocyclic compound having a carbazole ring as a partial structure of fused ring, carbazole derivatives, thiazole derivatives, benzimidazole derivatives, polydialkylfluorene derivatives may be used. Further, as the dopant material, quinacridone, coumarin, rubrene, perylene and their derivatives, a benzopyran derivative, a rhodamine derivative, an amino styryl derivative may be used. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer.

Further, the light emitting material may be a phosphorescent material. Phosphorescent materials as metal complexes of metals such as iridium and platinum may be used. Examples of the phosphorescent materials include green phosphorescent materials such as Ir(ppy)₃, blue phosphorescent materials such as FIrpic and FIr6, and red phosphorescent materials such as Btp₂Ir(acac). Here, as the hole injecting and transporting host material, 4,4'-di(N-carbazolyl)biphenyl (CBP), and carbazole derivatives such as TCTA and mCP may be used. As electron transporting host materials, p-bis(triphenylsilyl)benzene (UGH2), 2, 2',2"-(1,3,5-phenylene)-tris(1-phenyl-1H-benzimidazole) (TPBI) may be used. In this way, a high-performance organic EL device can be produced.

To avoid concentration quenching, the doping of the host material with the phosphorescent light emitting material should preferably be made by co-evaporation in a range of 1 to 30 weight percent with respect to the whole light emitting layer.

Further, as the light emitting material, it is also possible to use a material that emits delayed fluorescence. Such a material can be formed into a thin film by a known method such as a spin coating method or an inkjet method in addition to a vapor deposition method.

The hole blocking layer of the organic EL device of the present invention may be formed by using a hole blocking compound, such as phenanthroline derivatives such as bathocuproin (BCP), and the metal complexes of quinolinol derivatives such as aluminum(III)bis(2-methyl-8-quinolinate)-4-phenylphenolate (BAlq), various rare earth complexes, triazole derivatives, triazine derivatives, pyrimidine derivatives, oxadiazole derivatives, and benzazole derivatives. These materials may also serve as the material of the electron transport layer. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

The electron transport layer of the organic EL device of the present invention may be formed by using various metal complexes, triazole derivatives, triazine derivatives, pyrimidine derivatives, oxadiazole derivatives, pyridine derivatives, benzimidazole derivatives, benzazole derivatives, thiadiazole derivatives, anthracene derivatives, carbodiimide derivatives, quinoxaline derivatives, pyridoindole derivatives, phenanthroline derivatives, and silole derivatives in addition to the metal complexes of quinolinol derivatives such as Alq₃ and BAlq. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

The electron injection layer of the organic EL device of the present invention may be formed by using alkali metal salts such as lithium fluoride and cesium fluoride, alkaline earth metal salts such as magnesium fluoride, metal complexes of quinolinol derivatives such as lithium quinolinol, metal oxides such as aluminum oxide, and metals such as ytterbium (Yb), samarium (Sm), calcium (Ca), strontium (Sr), cesium (Cs). However, the electron injection layer may be omitted in the preferred selection of the electron transport layer and the cathode.

Further, in the electron injection layer or the electron transport layer, a material obtained by further N-doping a material which is commonly used for the layer with a metal such as cesium, or the like can be used.

The cathode of the organic EL device of the present invention may be made of an electrode material with a low work function such as aluminum, an alloy with an even lower work function such as a magnesium-silver alloy, magnesium calcium alloys, a magnesium-indium alloy, or an aluminum-magnesium alloy, or ITO or IZO.

The heterocyclic compound represented by the general formula (1), (1-a) or (1-b) is preferably used as the capping layer of the organic EL device of the present invention. These materials may be formed into a thin film by using a vapor deposition method or other known methods such as a spin coating method and an inkjet method.

The above description refers to an organic EL device with a top emission structure, but the present invention is not limited to this, and can also be applied to organic EL devices with bottom emission structures, and organic EL devices with dual emission structures that emit light from both the top and the bottom. In these cases, the electrode in the direction from which light is extracted from the light emitting device to the outside must be transparent or translucent.

### Examples

The following describes an embodiment of the present invention in more detail based on Examples. The present invention, however, is not restricted to the following Examples.

### Example 1

### <Synthesis of 2,5-bis{4-(phenanthrene-9-yl) phenyl} pyrimidine; Compound (1-7)>

To a reaction vessel purged with nitrogen, 5.0 g of 2,5-dichloropyrimidine, 50 mL of 1,4-dioxane, and 28.1 g of 4,4,5,5-tetramethyl-2-{4-(phenanthrene-9-yl)phenyl}-1,3,2-dioxaborolane were added, and then solution dissolved 21.4 g of tripotassium phosphate in 15 mL of purified water was added in this order, and the mixture was bubbling with nitrogen gas for 30 minutes. Then, 1.5 g of tris(dibenzylideneacetone)dipalladium, and 1.9 g of tricyclohexylphosphine were added thereto, and the mixture was stirred under heating to reflux for 12 hours. After the mixture was cooled to room temperature, 50 mL of methanol was added, the precipitated solid was collected by filtration. 1.0 L of chlorobenzene was added to the solid. After the mixture was heated to 100°C to dissolve the solid, 10 g of silica gel, and 10 g of activated clay were added and the mixture was stirred for 1 hour. The insoluble material was removed by hot filtration, the resulting filtrate was concentrated. The residue was recrystallized using chlorobenzene, whereby 12.6 g of a white powder of 2,5-bis{4-(phenanthrene-9-yl) phenyl} pyrimidine; Compound (1-7) (yield: 64%) was obtained.

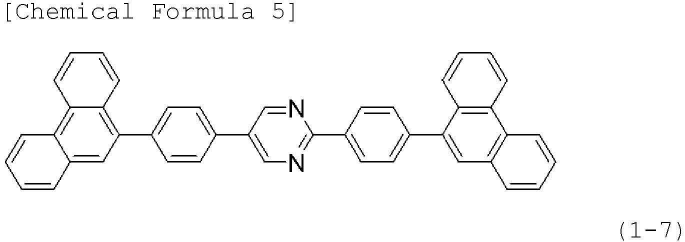

The structure of the obtained white powder was identified by NMR.

¹H-NMR (THF-d₈) detected 28 hydrogen signals, as follows.

δ (ppm) = 9.33 (2H), 8.80-8.94 (6H), 7.99-8.06 (6H), 7.60-7.85 (14H).

### Example 2

### <Synthesis of 2,5-bis{4-(phenanthrene-9-yl) phenyl} pyridine; Compound (1-41)>

The same operation as in Example 1 was performed except that 2,5-dichloropyrimidine was replaced with 2,5-dibromopyridine, whereby 11.5 g of a white powder of 2,5-bis{4-(phenanthrene-9-yl) phenyl} pyridine; Compound (1-41) (yield: 78%) was obtained.

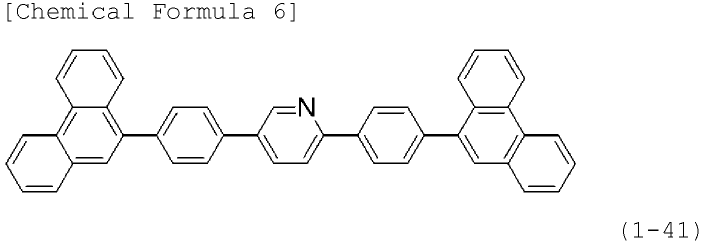

The structure of the obtained white powder was identified by NMR.

¹H-NMR (THF-d₈) detected 29 hydrogen signals, as follows.

δ (ppm) = 9.14 (1H), 8.81-8.89 (4H), 8.39-8.41 (2H), 8.22-8.23 (1H), 8.12-8.14 (1H), 7.95-8.01 (6H), 7.80 (2H), 7.56-7.79 (12H).

### Example 3

### <Synthesis of 5-{4-(dibenzofuran-3-yl) phenyl}-2-{4-(phenanthrene-9-yl) phenyl} pyrimidine; Compound (1-71)>

To a reaction vessel purged with nitrogen, 9.4 g of 2,5-dichloropyrimidine, 160 mL of toluene, 60 mL of ethanol, 20.0 g of 4,4,5,5-tetramethyl-2-{4-(phenanthrene-9-yl)phenyl}-1,3,2-dioxaborolane were added, and then solution dissolved 10.9 g of potassium carbonate in 40 mL of purified water were added in this order, and the mixture was bubbling with nitrogen gas for 30 minutes. Then, 0.6 g of tetrakis (triphenylphosphine) palladium (0) was added thereto, and the mixture was stirred under heating to reflux for 15 hours. After the mixture was cooled to room temperature and separated. After the organic layer was wash with water and saturated saline in this order, the organic layer was dried over anhydrous magnesium sulfate. After the desiccant was removed by filtration and the filtrate was concentrated. 200 mL of toluene was added to the residue, and the mixture was heated to 80°C, and then 10 g of silica gel, and 10 g of activated clay were added, the mixture was stirred for 1 hour. The insoluble matter was removed by filtration, and then the filtrate was concentrated. Acetone was added to the residue, dispersion washing was carried out, whereby 16.5 g of a white powder of 5-chloro-2-{4-(phenanthrene-9-yl) phenyl} pyrimidine (yield: 86%) was obtained.

The obtain 8.0 g of 5-chloro-2-{4-(phenanthrene-9-yl)phenyl}pyrimidine was added to a reaction vessel purged with nitrogen, and then 100 mL of 1,4-dioxane, 28.1 g of 3-{4-(4,4,5,5-tetramethyl-1,3,2-dioxaborolane-2-yl)phenyl}dibenzofuran, and solution dissolved 21.4 g of tripotassium phosphate in 15 mL of purified water were added thereto in this order, and the mixture was bubbling with nitrogen gas for 30 minutes. Then, 0.2 g of tris(dibenzylideneacetone)dipalladium, and 0.1 g of tricyclohexylphosphine were added thereto, and the mixture was stirred under heating to reflux for 13 hours. After the mixture was cooled to room temperature, the precipitated solid was collected by filtration. Methanol and water were added to the solid and reflux dispersion washing was performed for 1 hour. The solid was collected, and 750 mL of chlorobenzene was added to the solid. After the solid was heated to 100°C to dissolve, 8 g of silica gel, and 8 g of activated clay were added, and the mixture was stirred for 1 hour. The insoluble material was removed by hot filtration, the resulting filtrate was concentrated. The residue was recrystallized using chlorobenzene, whereby 10.5 g of a white powder of 5-{4-(dibenzofuran-3-yl) phenyl}-2-{4-(phenanthrene-9-yl) phenyl} pyrimidine; Compound (1-71) (yield: 72%) was obtained.

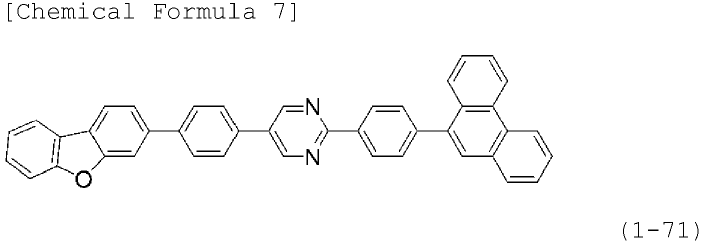

The structure of the obtained white powder was identified by NMR.

¹H-NMR (THF-d₈) detected 26 hydrogen signals, as follows.

δ (ppm) = 9.28 (2H), 8.76-8.92 (4H), 7.96-8.18 (9H), 7.36-7.86 (11H).

### Example 4

### <Synthesis of 5-{4-(phenanthrene-2-yl) phenyl}-2-{4-(phenanthrene-9-yl) phenyl} pyrimidine; Compound (1-74)>

The same operation as in Example 3 was performed except that 3-{4-(4,4,5,5-tetramethyl-1,3,2-dioxaborolane-2-yl)phenyl}dibenzofuran was replaced with 4,4,5,5-tetramethyl-2-{4-(phenanthrene-2-yl)phenyl}-1,3,2-dioxaborolane, whereby 8.6 g of a white powder of 5-{4-(phenanthrene-2-yl)phenyl}-2-{4-(phenanthrene-9-yl)phenyl}pyrimidine; Compound (1-74) (yield: 58%) was obtained.

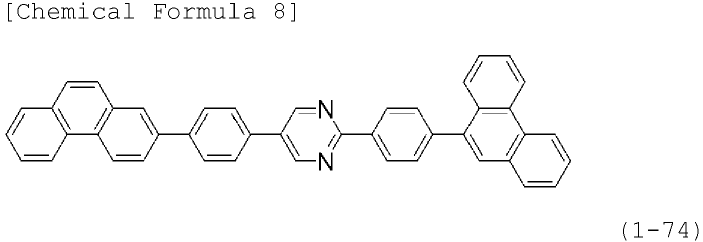

The structure of the obtained white powder was identified by NMR.

¹H-NMR (THF-d₈) detected 28 hydrogen signals, as follows.

δ (ppm) = 9.31 (2H), 8.78-8.93 (6H), 8.35-8.36 (1H), 7.91-8.13 (9H), 7.85-7.87 (2H), 7.60-7.76 (8H).

### Example 5

### <Synthesis of 2-{4-(phenanthrene-9-yl) phenyl}-5-{4-(1,10-phenanthroline-2-yl) phenyl} pyrimidine; Compound (1-137)>

The same operation as in Example 3 was performed except that 3-{4-(4,4,5,5-tetramethyl-1,3,2-dioxaborolane-2-yl)phenyl}dibenzofuran was replaced with 4,4,5,5-tetramethyl-2-{4-(1,10-phenanthroline-2-yl)phenyl}-1,3,2-dioxaborolane, whereby 7.5 g of a white powder of 2-{4-(phenanthrene-9-yl)phenyl}-5-{4-(1,10-phenanthroline-2-yl)phenyl}pyrimidine; Compound (1-137) (yield: 46.8%) was obtained.

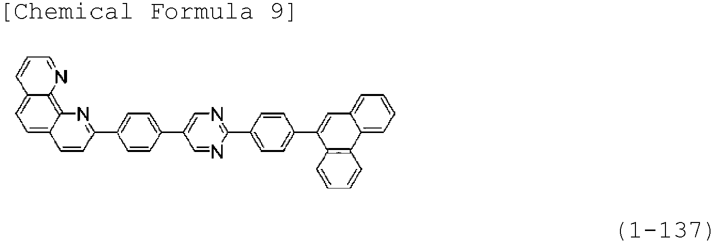

The structure of the obtained white powder was identified by NMR.

¹H-NMR (CDCl₃) detected 26 hydrogen signals, as follows.

δ (ppm) = 9.47 (1H), 8.82 (1H), 8.76 (1H), 8.47 (1H), 8.39 (2H), 8.31 (1H), 8.21 (1H), 8.17 (1H), 8.03 (1H), 8.00-7.89 (6H), 7.86 (1H), 7.78 (1H), 7.74 (2H), 7.70 (2H), 7.66 (1H), 7.61 (1H), 7.56-7.50 (2H).

### Example 6

### <Synthesis of 2,5-bis{4-(dibenzothiophene-4-yl) phenyl} pyrimidine; Compound (1-20)>

The same operation as in Example 1 was performed except that 4,4,5,5-tetramethyl-2-{4-(phenanthrene-9-yl)phenyl}-1,3,2-dioxaborolane was replaced with 4,4,5,5-tetramethyl-2-{4-(dibenzothiophene-4-yl)phenyl}-1,3,2-dioxaborolane, whereby 4.0 g of a white powder of 2,5-bis{4-(dibenzothiophene-4-yl)phenyl}pyrimidine; Compound (1-20) (yield: 40.0%) was obtained.

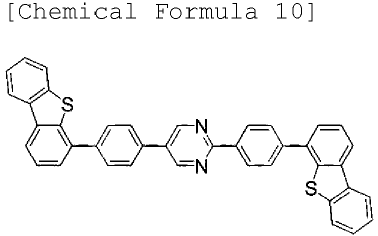

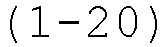

The structure of the obtained white powder was identified by NMR.

¹H-NMR (CDCl₃) detected 24 hydrogen signals, as follows.

δ (ppm) = 9.18 (2H), 8.69 (2H), 8.23 (4H), 7.95 (4H), 7.86 (4H), 7.64-7.54 (4H), 7.54-7.45 (4H).

### Example 7

### <Synthesis of 2,5-bis{4-(1,10-phenanthroline-2-yl) phenyl} pyrimidine; Compound (1-154)>

The same operation as in Example 1 was performed except that 4,4,5,5-tetramethyl-2-{4-(phenanthrene-9-yl)phenyl}-1,3,2-dioxaborolane was replaced with 4,4,5,5-tetramethyl-2-{4-(1,10-phenanthroline-2-yl)phenyl}-1,3,2-dioxaborolane, whereby 10.6 g of a white powder of 2,5-bis{4-(1,10-phenanthroline-2-yl)phenyl}pyrimidine; Compound (1-154) (yield: 59.5%) was obtained.

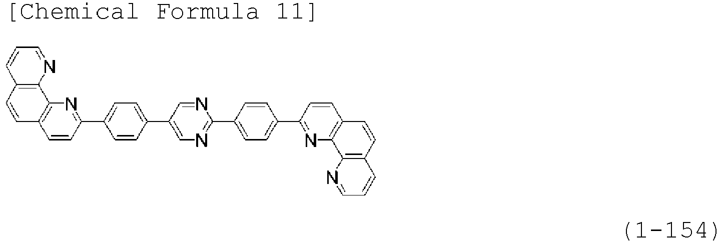

The structure of the obtained white powder was identified by NMR.

¹H-NMR (CDCl₃) detected 24 hydrogen signals, as follows.

δ (ppm) = 9.28 (1H), 8.80 (1H), 8.75 (1H), 8.50 (2H), 8.33 (1H), 8.27 (1H), 8.18 (1H), 8.03 (1H), 7.95-7.74 (8H), 7.72-7.54 (7H).

### Example 8

### <Synthesis of 5-(dibenzofuran-3-yl)-2-(9,9'-spirobi [9H] fluorene-2-yl) pyrimidine; Compound (1-89)>

To a reaction vessel purged with nitrogen, 30.0 g of 2,5-dichloropyrimidine, 66.0 g of 2-9,9'-spirobi[9H] fluorene boronic acid, 2.1 g of tetrakis(triphenylphosphine)palladium (0), and 38.0 g of potassium carbonate were added, the mixture in a toluene/ethanol/water mixed solvent was stirred under reflux overnight. After being allowed to cool, a toluene/water was added to the mixture, an organic layer was collected by extraction and liquid separation, and then concentrated to obtain a crude product. The obtained crude product was purified by column chromatography (support: silica gel, eluent: dichloromethane/n-heptane), whereby 39.0 g of a white powder of 5-chloro-2-(9,9'-spirobi[9H] fluorene-2-yl) pyrimidine (yield: 49.6%) was obtained.

The obtained 9.5 g of 5-chloro-2-(9,9'-spirobi[9H]fluorene-2-yl)pyrimidine was added to a reaction vessel purged with nitrogen, and 5.2 g (dibenzofuran-3-yl)boronicacid, 6.1 g of tripotassium phosphate, 0.2 g of tris(dibenzylideneacetone) dipalladium(0), 0.1 g of tricyclohexylphosphine, 100 mL of 1,4-dioxane, and 30 mL of purified water were added thereto, and the mixture was stirred under heating to reflux overnight. After being allowed to cool, a solid precipitated by adding methanol was collected by filtration to obtain a crude product. The obtained crude product was purified by crystallization using a mixed solvent of chlorobenzene/acetone, whereby 8.4 g of a white powder of 5-(dibenzofuran-3-yl)-2-(9,9'-spirobi[9H] fluorene-2-yl) pyrimidine; Compound (1-89) (yield: 68.0%) was obtained.

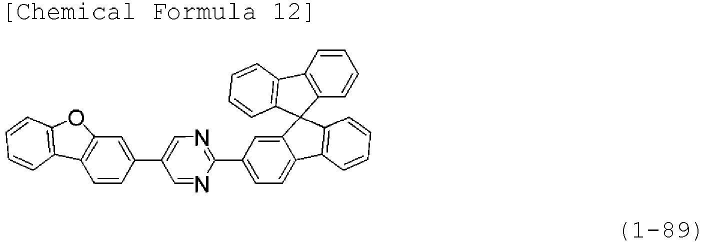

The structure of the obtained white powder was identified by NMR.

¹H-NMR (CDCl₃) detected 24 hydrogen signals, as follows.

δ (ppm) = 8.97 (2H), 8.61 (1H), 8.04 (1H), 8.00 (1H), 7.98 (1H), 7.92 (1H), 7.90 (1H), 7.88 (2H), 7.74 (1H), 7.59 (1H), 7.53 (1H), 7.49 (1H), 7.39 (4H), 7.14 (1H), 7.11 (2H), 6.79 (2H), 6.74 (1H).

### Example 9

### <Synthesis of 5-{4-(naphthalene-1-yl) phenyl}-2-(9,9'-spirobi[9H] fluorene-2-yl) pyrimidine; Compound (1-142)>

The same operation as in Example 8 was performed except that (dibenzofuran-3-yl) boronic acid was replaced with 4-(naphthalene-1-yl) phenylboronic acid, whereby 9.8 g of a white powder of 5-{4- (naphthalene-1-yl) phenyl}-2-(9,9'-spirobi[9H] fluorene-2-yl) pyrimidine; Compound (1-142) (yield: 71.0%) was obtained.

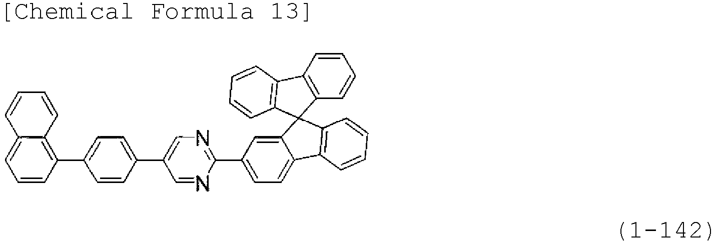

The structure of the obtained white powder was identified by NMR.

¹H-NMR (CDCl₃) detected 28 hydrogen signals, as follows.

δ (ppm) = 8.98 (2H), 8.62 (1H), 8.01 (1H), 7.90 (7H), 7.65 (4H), 7.58-7.34 (7H), 7.14 (1H), 7.11 (2H), 6.79 (2H), 6.74 (1H).

### Example 10

### <Synthesis of 5-{4-(naphthalene-2-yl) phenyl}-2-(9,9'-spirobi[9H] fluorene-2-yl) pyrimidine; Compound (1-143) >

The same operation as in Example 8 was performed except that (dibenzofuran-3-yl) boronic acid was replaced with 4-(naphthalene-2-yl) phenylboronic acid, whereby 10.6 g of a white powder of 5-{4-(naphthalene-2-yl) phenyl}-2-(9,9'-spirobi[9H] fluorene-2-yl) pyrimidine; Compound (1-143) (yield: 76.3%) was obtained.

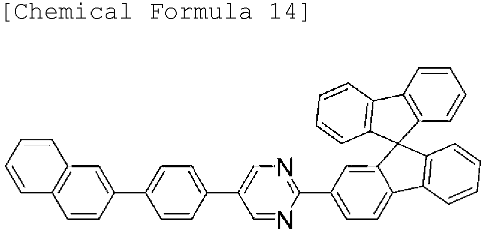

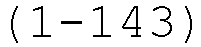

The structure of the obtained white powder was identified by NMR.

¹H-NMR (CDCl₃) detected 28 hydrogen signals, as follows.

δ (ppm) = 8.95 (2H), 8.60 (1H), 8.07 (1H), 8.00 (1H), 7.96-7.81 (9H), 7.76 (1H), 7.67 (2H), 7.51 (2H), 7.38 (3H), 7.14 (1H), 7.11 (2H), 6.79 (2H), 6.74 (1H).

### Example 11

### <Synthesis of 5-{4-(benzothiophene-2-yl) phenyl}-2-(9,9'-spirobi[9H] fluorene-2-yl) pyrimidine; Compound (1-146)>

The same operation as in Example 8 was performed except that (dibenzofuran-3-yl)boronicacid was replaced with 4,4,5,5-tetramethyl-2-{4-(benzothiophene-2-yl)phenyl}-1,3,2-dioxaborolane, whereby 9.4 g of a white powder of 5-{4-(benzothiophene-2-yl)phenyl}-2-(9,9'-spirobi[9H]fluorene-2-yl)pyrimidine; Compound (1-146) (yield: 70.0%) was obtained.

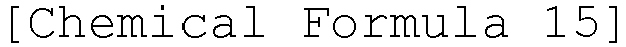

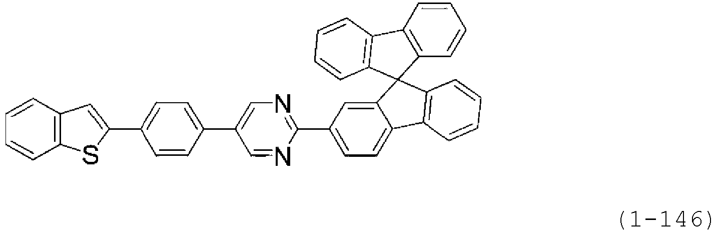

The structure of the obtained white powder was identified by NMR.

¹H-NMR (CDCl₃) detected 26 hydrogen signals, as follows.

δ (ppm) = 8.93 (2H), 8.60 (1H), 8.00 (1H), 7.92 (1H), 7.90-7.81 (6H), 7.79 (1H), 7.62 (3H), 7.43-7.30 (5H), 7.14 (1H), 7.11 (2H), 6.78 (2H), 6.74 (1H).

### Example 12

### <Synthesis of 5-{4-(benzoxazole-2-yl) phenyl}-2-(9,9'-spirobi[9H] fluorene-2-yl) pyrimidine; Compound (1-147)>

The same operation as in Example 8 was performed except that (dibenzofuran-3-yl)boronicacid was replaced with 4,4,5,5-tetramethyl-2-{4-(benzoxazole-2-yl)phenyl}-1,3,2-dioxaborolane, whereby 12.5 g of a white powder of 5-{4-(benzoxazole-2-yl)phenyl}-2-(9,9'-spirobi[9H]fluorene-2-yl)pyrimidine; Compound 1-147 (yield: 91.2%) was obtained.

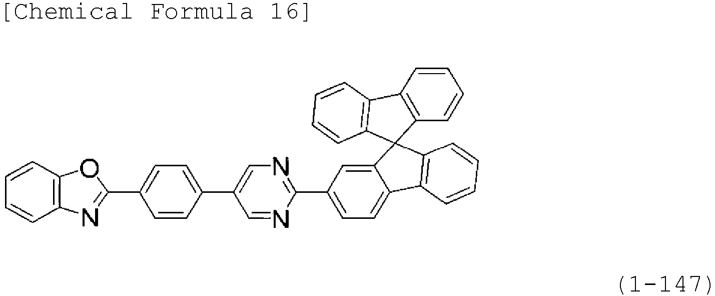

The structure of the obtained white powder was identified by NMR.

¹H-NMR (CDCl₃) detected 25 hydrogen signals, as follows.

δ (ppm) = 8.96 (2H), 8.61 (1H), 8.37 (2H), 8.00 (1H), 7.92 (1H), 7.90 (1H), 7.88 (2H), 7.80 (1H), 7.72 (2H), 7.61 (1H), 7.42-7.35 (5H), 7.15 (1H), 7.11 (2H), 6.78 (2H), 6.74 (1H).

### Example 13

### <Synthesis of 5-{4-(benzothiazole-2-yl) phenyl}-2-(9,9'-spirobi[9H] fluorene-2-yl)pyrimidine; Compound (1-148)>

The same operation as in Example 8 was performed except that (dibenzofuran-3-yl)boronicacid was replaced with 4,4,5,5-tetramethyl-2-{4-(benzothiazole-2-yl)phenyl}-1,3,2-dioxaborolane, whereby 11.5 g of a white powder of 5-{4-(benzothiazole-2-yl)phenyl}-2-(9,9'-spirobi[9H]fluorene-2-yl)pyrimidine; Compound (1-148) (yield: 81.6%) was obtained.

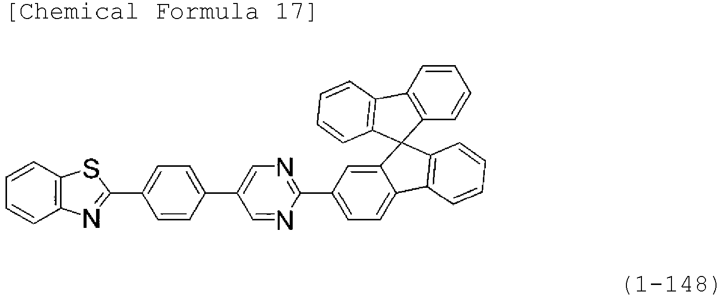

The structure of the obtained white powder was identified by NMR.

¹H-NMR (CDCl₃) detected 25 hydrogen signals, as follows.

δ (ppm) = 8.95 (2H), 8.61 (1H), 8.22 (2H), 8.10 (1H), 8.00 (1H), 7.92 (2H), 7.89 (1H), 7.88 (2H), 7.69 (2H), 7.52 (1H), 7.44-7.35 (4H), 7.15 (1H), 7.11 (2H), 6.78 (2H), 6.75 (1H).

### Example 14

### <Synthesis of 5-{4-(9-phenyl-carbazole-3-yl)phenyl}-2-(9,9'-spirobi[9H]fluorene-2-yl)pyrimidine; Compound (1-149)>

The same operation as in Example 8 was performed except that (dibenzofuran-3-yl)boronicacid was replaced with 4,4,5,5-tetramethyl-2-{4-(9-phenyl-carbazole-3-yl)phenyl}-1,3,2-dioxaborolane, whereby 10.1 g of a pale yellow powder of 5-{4-(9-phenyl-carbazole-3-yl)phenyl}-2-(9,9'-spirobi[9H]fluorene-2-yl)pyrimidine; Compound (1-149) (yield: 62.9%) was obtained.

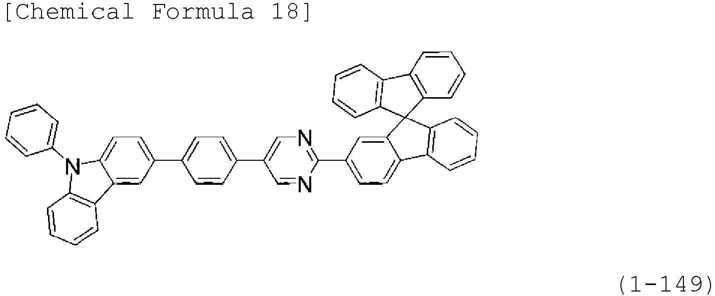

The structure of the obtained pale-yellow powder was identified by NMR.

¹H-NMR (CDCl₃) detected 33 hydrogen signals, as follows.

δ (ppm) = 8.97 (2H), 8.61 (1H), 8.38 (1H), 8.20 (1H), 8.00 (1H), 7.92 (1H), 7.90 (1H), 7.88 (2H), 7.85 (2H), 7.70-7.56 (7H), 7.50 (1H), 7.48 (1H), 7.43 (2H), 7.38 (3H), 7.32 (1H), 7.14 (1H), 7.11 (2H), 6.79 (2H), 6.74 (1H).

### Example 15

### <Synthesis of 2-{4-(phenanthrene-9-yl) phenyl}-5-{3,5-bis(naphthalene-2-yl) phenyl} pyrimidine; Compound (1-150) >

The same operation as in Example 3 was performed except that 3-{4-(4,4,5,5-tetramethyl-1,3,2-dioxaborolane-2-yl)phenyl}dibenzofuran was replaced with 4,4,5,5-tetramethyl-2-{3,5-bis(naphthalene-2-yl)phenyl}-1,3,2-dioxaborolane, whereby 1.8 g of a white powder of 2-{4-(phenanthrene-9-yl)phenyl}-5-{3,5-bis(naphthalene-2-yl)phenyl}pyrimidine; Compound (1-150) (yield: 22.2%) was obtained.

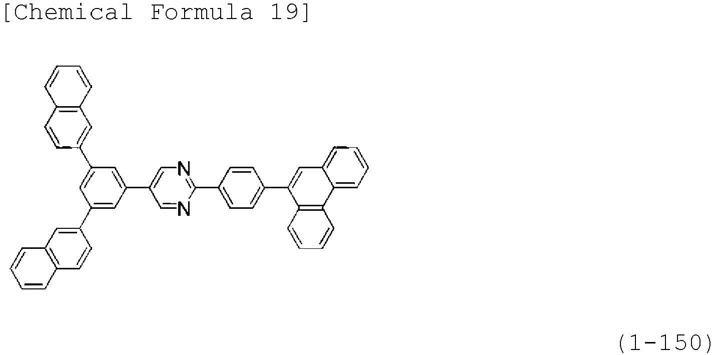

The structure of the obtained white powder was identified by NMR.

¹H-NMR (CDCl₃) detected 32 hydrogen signals, as follows.

δ (ppm) = 9.25 (2H), 8.81 (1H), 8.75 (1H), 8.69 (2H), 8.22 (2H), 8.16 (1H), 8.04-7.88 (12H), 7.77 (1H), 7.74 (2H), 7.70 (2H), 7.64 (1H), 7.60-7.51 (5H).

### Example 16

### <Synthesis of 2-{4-(phenanthrene-9-yl) phenyl}-5-{3,5-bis(quinoline-3-yl) phenyl} pyrimidine; Compound (1-151) >

The same operation as in Example 3 was performed except that 3-{4-(4,4,5,5-tetramethyl-1,3,2-dioxaborolane-2-yl)phenyl}dibenzofuran was replaced with 4,4,5,5-tetramethyl-2-{3,5-bis(quinoline-3-yl)phenyl}-1,3,2-dioxaborolane, whereby 4.3 g of a white powder of 2-{4-(phenanthrene-9-yl)phenyl}-5-{3,5-bis(quinoline-3-yl)phenyl}pyrimidine; Compound 1-151 (yield: 47.6%) was obtained.

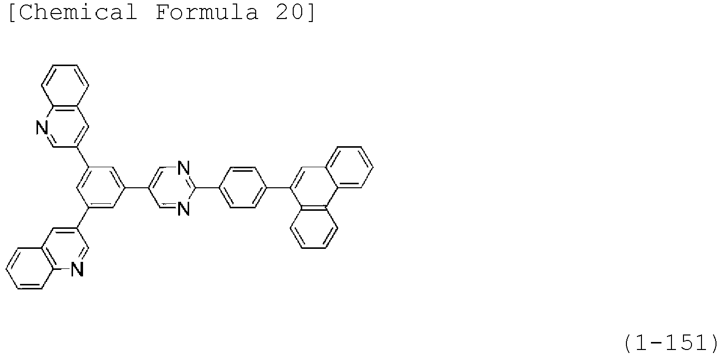

The structure of the obtained white powder was identified by NMR.

¹H-NMR (CDCl₃) detected 30 hydrogen signals, as follows.

δ (ppm) = 9.34 (2H), 9.24 (2H), 8.80 (1H), 8.74 (1H), 8.69 (2H), 8.49 (2H), 8.22 (2H), 8.13 (1H), 8.03 (2H), 8.00 (1H), 7.97 (2H), 7.93 (1H), 7.80 (2H), 7.77 (1H), 7.74 (2H), 7.72-7.61 (5H), 7.57 (1H).

### Example 17

### <Synthesis of 2-{4-(phenanthrene-9-yl) phenyl}-5-{3,5-bis(naphthalene-2-yl) phenyl} pyridine; Compound (1-152) >

To a reaction vessel purged with nitrogen, 9.3 g of 2,5-dichloropyridine, 10.0 g of 4,4,5,5-tetramethyl-2-{4-(phenanthrene-9-yl) phenyl}-1,3,2-dioxaborolane, 0.8 g of tetrakis(triphenylphosphine) palladium (0), 10.9 g of potassium carbonate were added, the mixture in a toluene/ethanol/water mixed solvent was stirred under reflux for 5 hours. After being allowed to cool, a toluene/water was added to the mixture, an organic layer was collected by extraction and liquid separation, and then concentrated to obtain a crude product. The obtained crude product was purified by column chromatography (support: silica gel, eluent: toluene/ n-heptane), whereby 9.6 g of a white powder of 5-chloro-2-{4-(phenanthrene-9-yl) phenyl} pyridine (yield: 81.5%) was obtained.

The obtained 5.0 g of 5-chloro-2-{4-(phenanthrene-9-yl)phenyl}pyridine was added to a reaction vessel purged with nitrogen, and 6.7 g of 4,4,5,5-tetramethyl-2-{3,5-bis(naphthalene-2-yl)phenyl}-1,3,2-dioxaborolane, 0.4 g of tetrakis(triphenylphosphine)palladium (0), 2.5 g of potassium carbonate were added, the mixture in a toluene/ethanol/water mixed solvent was stirred under reflux overnight. After being allowed to cool, a solid precipitated by adding methanol was collected by filtration to obtain a crude product. The obtained crude product was purified by recrystallizing with a toluene solvent, whereby 6.7 g of a white powder of 2-{4-(phenanthrene-9-yl) phenyl}-5-{3,5-bis(naphthalene-2-yl) phenyl} pyridine; Compound (1-152) (yield: 83.3%) was obtained.

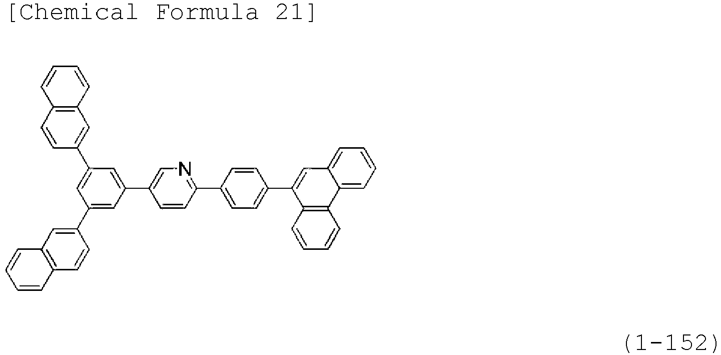

The structure of the obtained white powder was identified by NMR.

¹H-NMR (CDCl₃) detected 33 hydrogen signals, as follows.

δ (ppm) = 9.17 (1H), 8.80 (1H), 8.75 (1H), 8.25 (2H), 8.22 (2H), 8.18 (1H), 8.12 (1H), 8.04-7.95 (8H), 7.95-7.88 (5H), 7.76 (1H), 7.74-7.66 (4H), 7.64 (1H), 7.60-7.51 (5H).

### Example 18

### <Synthesis of 2-{4-(phenanthrene-9-yl) phenyl}-5-{3,5-bis(quinoline-3-yl) phenyl} pyridine; Compound (1-153) >

The same operation as in Example 17 was performed except that 4,4,5,5-tetramethyl-2-{3,5-bis(naphthalene-2-yl)phenyl}-1,3,2-dioxaborolane was replaced with 4,4,5,5-tetramethyl-2-{3,5-bis(quinoline-3-yl)phenyl}-1,3,2-dioxaborolane, whereby 5.3 g of a white powder of 2-{4-(phenanthrene-9-yl)phenyl}-5-{3,5-bis(quinoline-3-yl)phenyl}pyridine; Compound (1-153) (yield: 73.0%) was obtained.

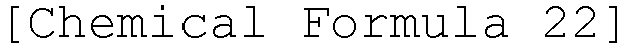

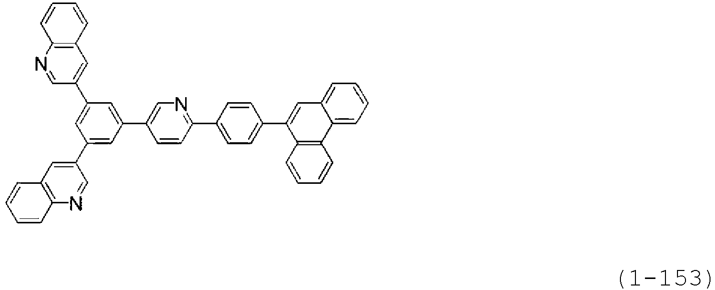

The structure of the obtained white powder was identified by NMR.

¹H-NMR (CDCl₃) detected 31 hydrogen signals, as follows.

δ (ppm) = 9.34 (2H), 9.16 (2H), 8.80 (1H), 8.74 (1H), 8.48 (2H), 8.28 (2H), 8.21 (2H), 8.17 (1H), 8.08 (1H), 8.05 (2H), 8.00 (2H), 7.96 (2H), 7.92 (1H), 7.79 (2H), 7.74-7.60 (7H), 7.56 (1H).

### Example 19

The glass transition points (Tg) and the melting points of the heterocyclic compound represented by the general formula (1) were determined with a high-sensitivity differential scanning calorimeter (DSC3100SA produced by Bruker AXS). The measurement results are summarized in Table 1.

**[Table 1]**

| | Glass transition point (Tg) | Melting point |
|---|---|---|
| Compound(1-7) | 116°C | 296°C |
| Compound(1-41) | not observed | 314°C |
| Compound(1-71) | not observed | 269°C |
| Compound(1-74) | not observed | 309°C |
| Compound(1-137) | 127°C | 245°C |
| Compound(1-20) | not observed | 301°C |
| Compound(1-154) | 125°C | 245°C |
| Compound(1-89) | 143°C | 329°C |
| Compound(1-142) | 145°C | 328°C |
| Compound(1-143) | not observed | 357°C |
| Compound(1-146) | not observed | 366°C |
| Compound(1-147) | not observed | 377°C |
| Compound(1-148) | not observed | 379°C |
| Compound(1-149) | 170°C | 400°C |
| Compound(1-150) | 120°C | not observed |
| Compound(1-151) | 137°C | not observed |
| Compound(1-152) | 114°C | not observed |
| Compound(1-153) | 128°C | not observed |

As shown above, the heterocyclic compounds represented by the general formula (1) of the present invention have glass transition points (Tg) of 100°C or higher, or for which the glass transition point (Tg) is not observed, demonstrating that the compounds have a stable thin-film state.

### Example 20

An 80 nm-thick vapor-deposited film was fabricated on a silicon substrate using the heterocyclic compound represented by the general formula (1), and the refractive index n at wavelengths of 450 nm and 750 nm was measured with a spectrometer (F10-RT-UV produced by Filmetrics). For comparison, comparative compound (2-1) of the following structural formula and Alq₃ were also measured. The measurement results are summarized in Table 2.(2-1) is known from i.a. WO2015/190400

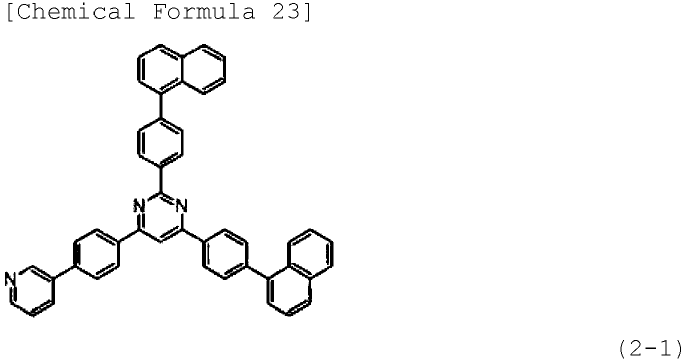

**[Table 2]**

| | Refractive index n (*λ* : 450nm) | Refractive index n (*λ* : 750nm) |
|---|---|---|
| Compound(1-7) | 2.10 | 1.91 |
| Compound(1-41) | 2.05 | 1.88 |
| Compound(1-71) | 2.10 | 1.89 |
| Compound(1-74) | 2.13 | 1.92 |
| Compound(1-137) | 2.04 | 1.82 |
| Compound(1-20) | 2.05 | 1.86 |
| Compound(1-154) | 2.08 | 1.89 |
| Compound(1-89) | 2.05 | 1.83 |
| Compound(1-142) | 2.01 | 1.83 |
| Compound(1-143) | 2.05 | 1.84 |
| Compound(1-146) | 2.10 | 1.85 |
| Comp.ound(1-147) | 2.08 | 1.85 |
| Compound(1-148) | 2.11 | 1.86 |
| Compound(1-149) | 2.08 | 1.85 |
| Comp.ound(1-150) | 2.06 | 1.89 |
| Compound(1-151) | 2.08 | 1.91 |
| Compound(1-152) | 2.05 | 1.88 |
| Compound(1-153) | 2.08 | 1.91 |
| Alq₃ | 1.88 | 1.73 |
| Comperative Compound(2-1) | 1.93 | 1.81 |

As shown above, the refractive index n of the heterocyclic compound represented by the general formula (1) of the present invention has the value equal to or higher than the refractive index of Alq₃ and the comparative compound (2-1) between wavelengths of 450 nm and 750 nm. This indicates that an improvement in light extraction efficiency in the organic EL device can be expected by using the heterocyclic compound represented by the general formula (1) of the present invention as a constitutive material of the capping layer.

### Example 21

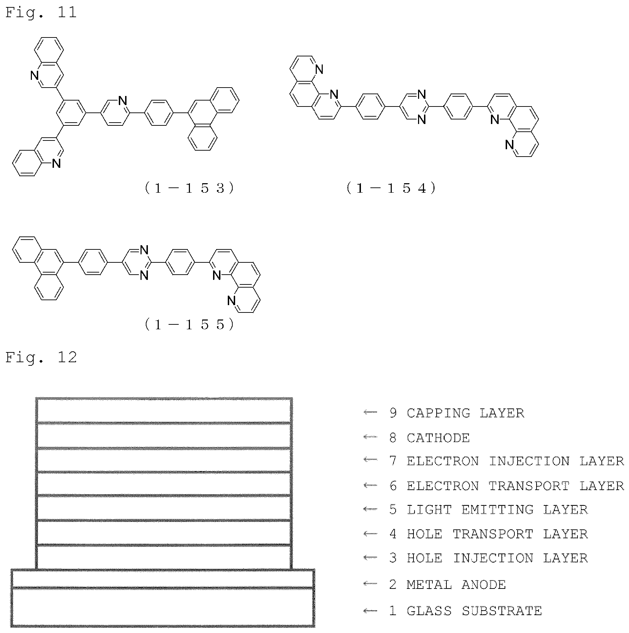

An organic EL device, as shown in Fig. 12, was fabricated by vapor-depositing a hole injection layer 3, a hole transport layer 4, a light emitting layer 5, an electron transport layer 6, an electron injection layer 7, a cathode 8, and a capping layer 9 in this order on a glass substrate 1 on which a reflective ITO electrode as a metal anode 2 had been formed beforehand.

Specifically, as the metal anode 2, an ITO film with a thickness of 50 nm, a silver alloy reflective film with a thickness of 100 nm, and an ITO film with a thickness of 5 nm were deposited on the glass substrate 1 in this order. After ultrasonic cleaning in isopropyl alcohol for 20 minutes, the assembly was dried on a hot plate heated to 250°C for 10 minutes. After UV ozone treatment for 2 minutes, the glass substrate with ITO was installed in a vacuum vapor deposition apparatus, and the pressure was reduced to 0.001 Pa or lower. Subsequently, as the hole injection layer 3 covering the metal anode 2, an electron acceptor (Acceptor-1) of the structural formula below and a compound (3-1) of the structural formula below were formed in a film thickness of 10 nm by dual vapor deposition at a vapor deposition rate ratio Acceptor-1:compound (3-1) = 3:97. The hole transport layer 4 was formed on the hole injection layer 3 by forming the compound (3-1) of the structural formula below in a film thickness of 140 nm. The light emitting layer 5 was formed on the hole transport layer 4 in a film thickness of 20 nm by dual vapor deposition of a compound (3-2) of the structural formula below and a compound (3-3) of the structural formula below at a vapor deposition rate ratio of compound (3-2):compound (3-3) = 5:95. The electron transport layer 6 was formed on the light emitting layer 5 in a film thickness of 30 nm by dual vapor deposition of a compound (3-4) of the structural formula below and a compound (3-5) of the structural formula below at a vapor deposition rate ratio of compound (3-4):compound (3-5) = 50:50. The electron injection layer 7 was formed on the electron transport layer 6 by forming lithium fluoride in a film thickness of 1 nm. On the electron injection layer 7, a magnesium silver alloy was formed as the cathode 8 with a thickness of 12 nm. Finally, the compound (1-7) of Example 1 was formed as the capping layer 9 with a film thickness of 60 nm. The characteristics of the organic EL device thus fabricated were measured in the atmosphere at an ordinary temperature. Table 3 summarizes the results of the emission characteristics measurements performed by applying a DC voltage to the fabricated organic EL device.

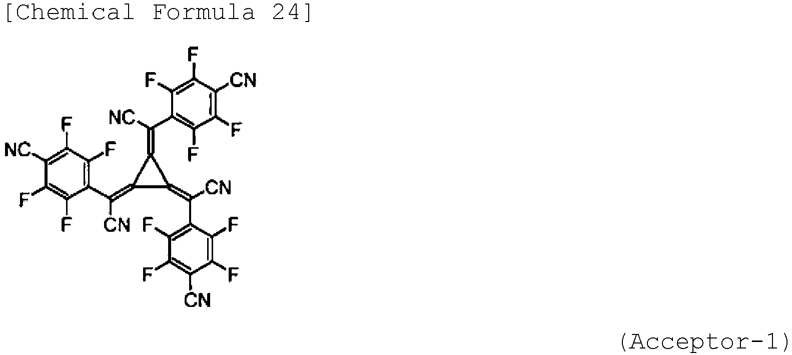

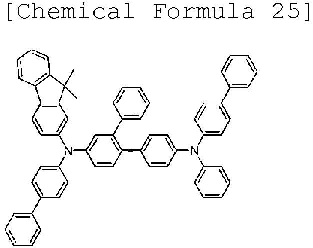

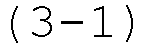

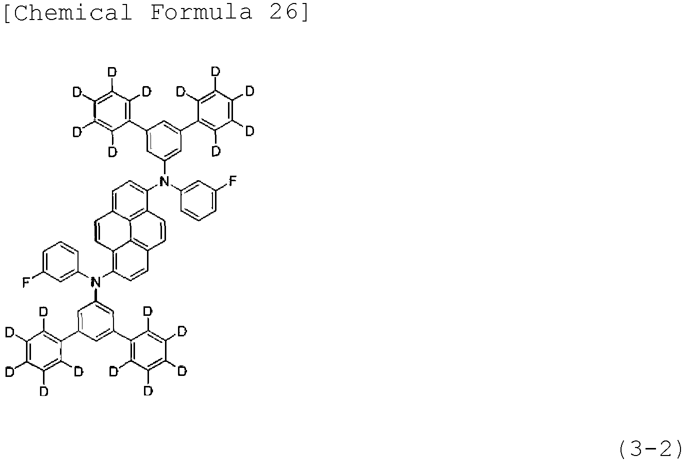

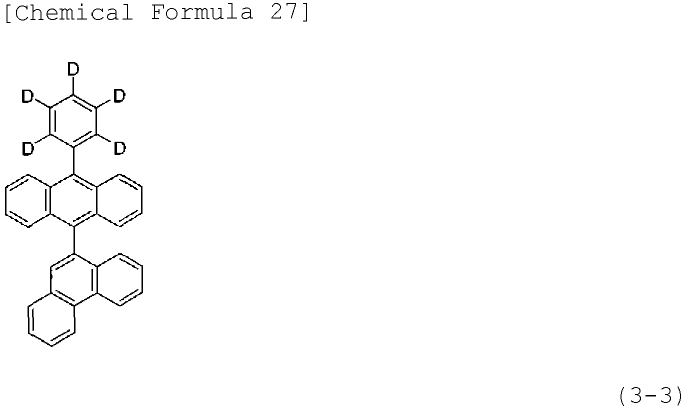

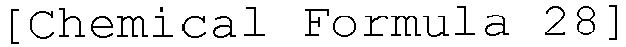

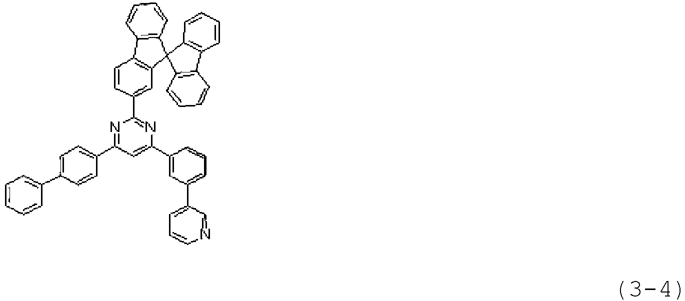

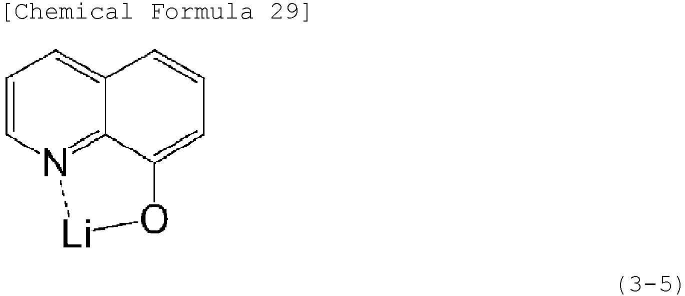

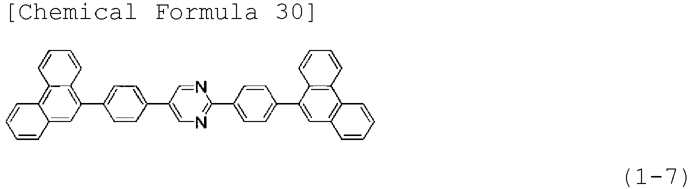

### Example 22

An organic EL device was fabricated under the same conditions as in Example 21 except that the capping layer 9 was formed by using the compound (1-41) of Example 2 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

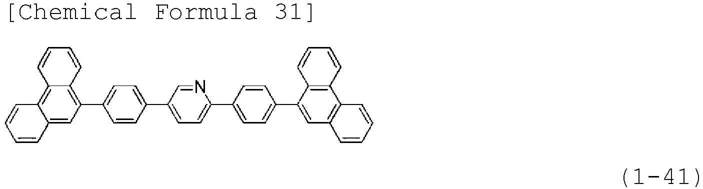

### Example 23

An organic EL device was fabricated under the same conditions as in Example 21 except that the capping layer 9 was formed by using the compound (1-71) of Example 3 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

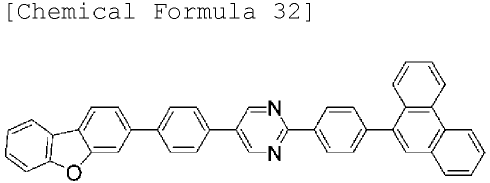

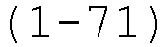

### Example 24

An organic EL device was fabricated under the same conditions as in Example 21 except that the capping layer 9 was formed by using the compound (1-74) of Example 4 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

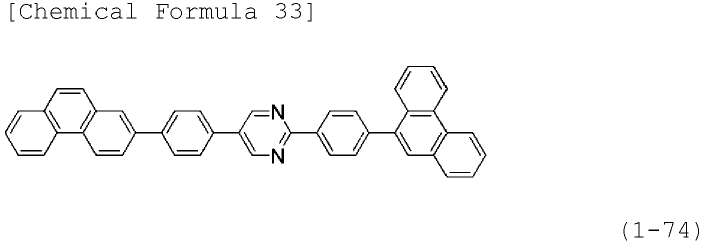

### Example 25

An organic EL device was fabricated under the same conditions as in Example 21 except that the capping layer 9 was formed by using the compound (1-137) of Example 5 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

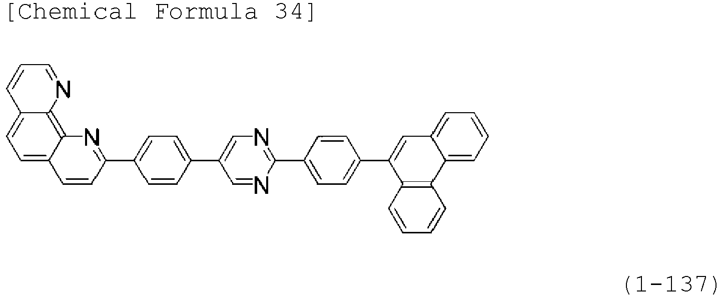

### Example 26

An organic EL device was fabricated under the same conditions as in Example 21 except that the capping layer 9 was formed by using the compound (1-20) of Example 6 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

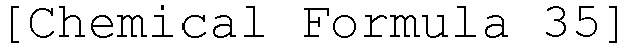

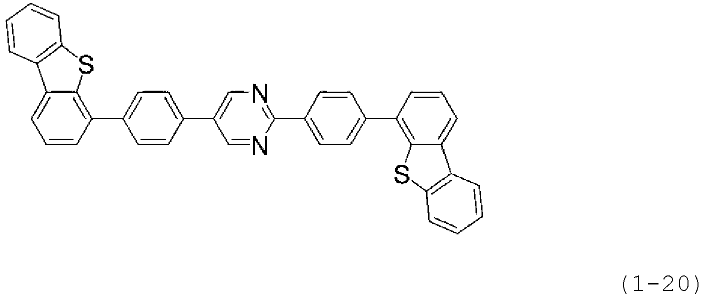

### Example 27

An organic EL device was fabricated under the same conditions as in Example 21 except that the capping layer 9 was formed by using the compound (1-154) of Example 7 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

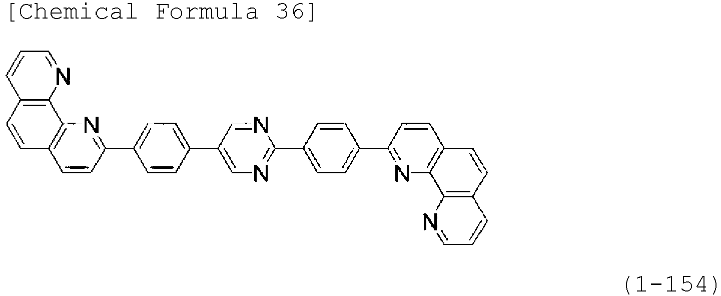

### Example 28

An organic EL device was fabricated under the same conditions as in Example 21 except that the capping layer 9 was formed by using the compound (1-89) of Example 8 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

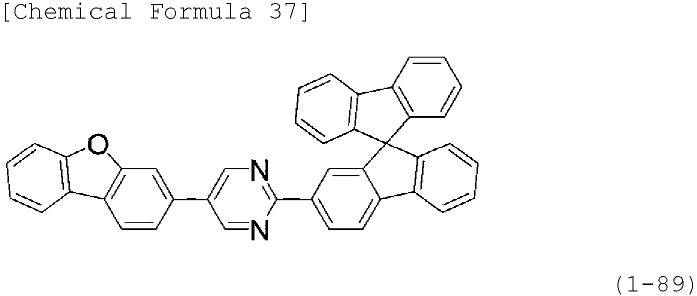

### Example 29

An organic EL device was fabricated under the same conditions as in Example 21 except that the capping layer 9 was formed by using the compound (1-142) of Example 9 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

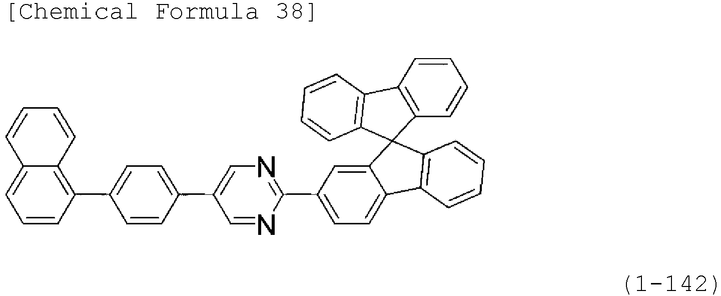

### Example 30

An organic EL device was fabricated under the same conditions as in Example 21 except that the capping layer 9 was formed by using the compound (1-143) of Example 10 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

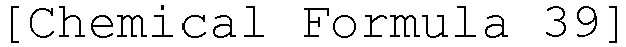

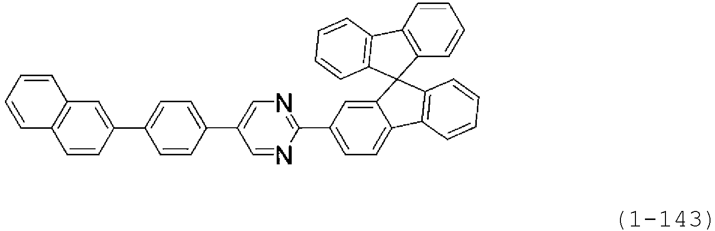

### Example 31

An organic EL device was fabricated under the same conditions as in Example 21 except that the capping layer 9 was formed by using the compound (1-146) of Example 11 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

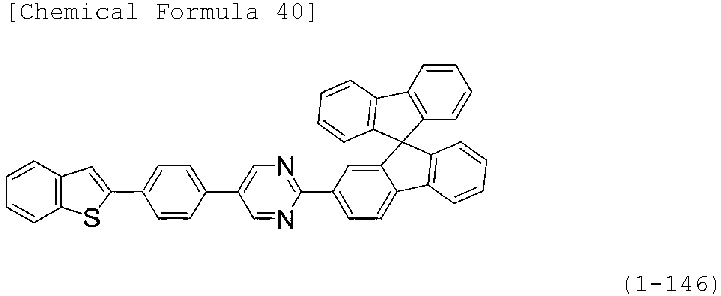

### Example 32

An organic EL device was fabricated under the same conditions as in Example 21 except that the capping layer 9 was formed by using the compound (1-147) of Example 12 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

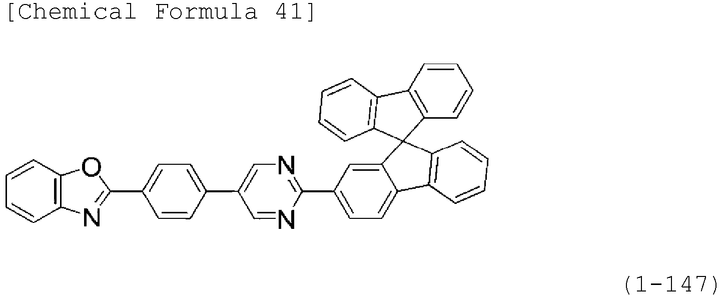

### Example 33

An organic EL device was fabricated under the same conditions as in Example 21 except that the capping layer 9 was formed by using the compound (1-148) of Example 13 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

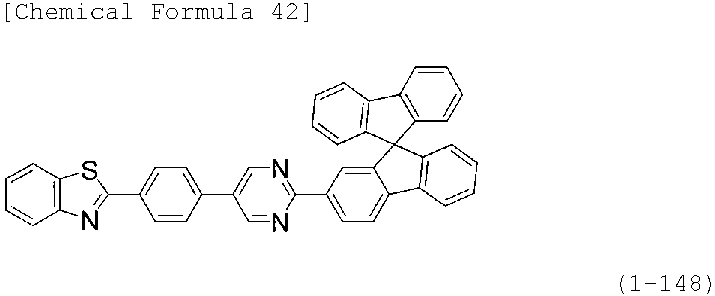

### Example 34

An organic EL device was fabricated under the same conditions as in Example 21 except that the capping layer 9 was formed by using the compound (1-149) of Example 14 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

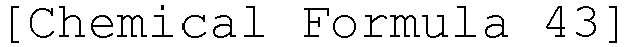

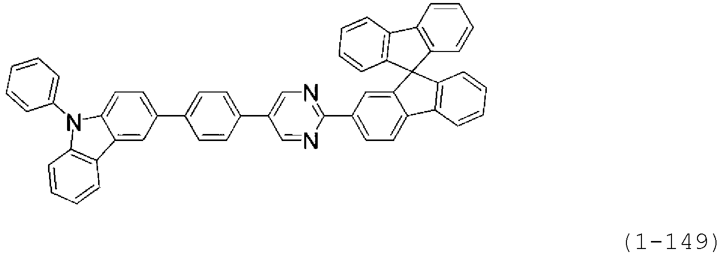

### Example 35

An organic EL device was fabricated under the same conditions as in Example 21 except that the capping layer 9 was formed by using the compound (1-150) of Example 15 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

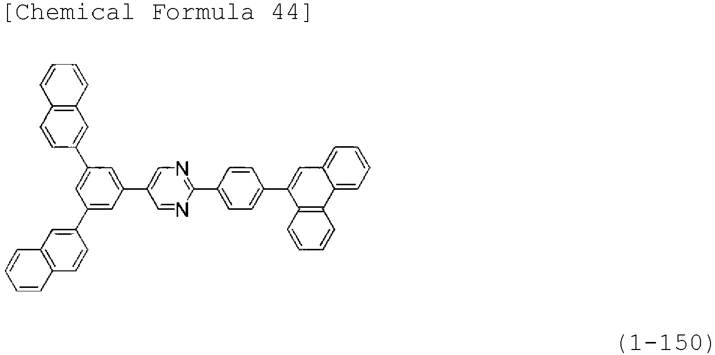

### Example 36

An organic EL device was fabricated under the same conditions as in Example 21 except that the capping layer 9 was formed by using the compound (1-151) of Example 16 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

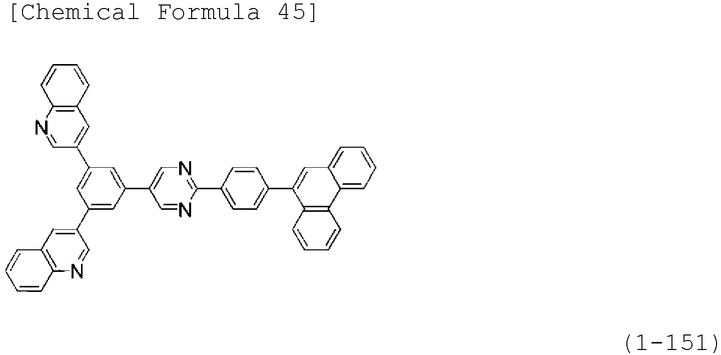

### Example 37

An organic EL device was fabricated under the same conditions as in Example 21 except that the capping layer 9 was formed by using the compound (1-152) of Example 17 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

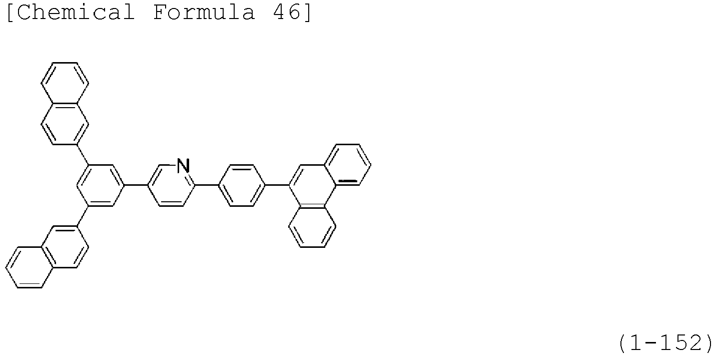

### Example 38

An organic EL device was fabricated under the same conditions as in Example 21 except that the capping layer 9 was formed by using the compound (1-153) of Example 18 instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

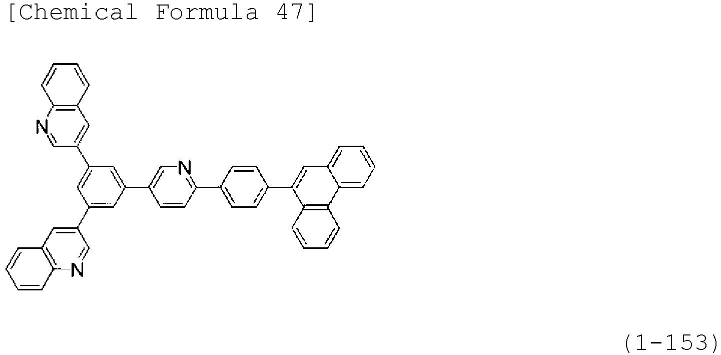

### Comparative Example 1

For comparison, an organic EL device was fabricated under the same conditions as in Example 21 except that the capping layer 9 was formed by using Alq₃ instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

### Comparative Example 2

For comparison, an organic EL device was fabricated under the same conditions as in Example 21 except that the capping layer 9 was formed by using a comparative compound (2-1) instead of the compound (1-7) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

Table 3 summarizes the results of the device lifetime measurements performed with the organic EL devices fabricated in Examples 21 to 38 and Comparative Examples 1 to 2. The device lifetime was measured as the time (attenuation to 95%) taken for the luminance to decay to 95%, with the initial luminance as 100%, when carrying out constant current drive of 10 mA/cm².

**[Table 3]**

| | Capping layer | Voltage [V] | Luminance [cd/m²] | Luminous efficiency [cd/A] | Power efficiency [lm/W] | Device lifetime attenuation to 95% |
|---|---|---|---|---|---|---|
| | | (@10mA/cm²) | (@10mA/cm²) | (@10mA/cm²) | (@10mA/cm²) | |
| Example21 | Compound(1-7) | 3.62 | 786 | 7.86 | 6.83 | 158hr. |
| Example22 | Compound(1-41) | 3.61 | 768 | 7.68 | 6.69 | 153hr. |
| Example23 | Compound(1-71) | 3.66 | 769 | 7.88 | 6.59 | 166hr. |
| Example24 | Compound(1-74) | 3.64 | 840 | 8.40 | 7.26 | 162hr. |
| Example25 | Compound(1-137) | 3.66 | 759 | 7.59 | 6.53 | **153hr.** |
| Example26 | Compound(1-20) | 3.64 | 779 | 7.79 | 6.72 | 171hr. |
| Example27 | Compound(1-154) | 3.67 | 758 | 7.58 | 6.49 | 166hr. |
| Example28 | Compound(1-89) | 3.65 | 783 | 7.83 | 6.75 | 159hr. |
| Example29 | Compound(1-142) | 3.60 | 825 | 8.25 | 7.20 | 174hr. |
| Example30 | Compound(1-143) | 3.61 | 806 | 8.06 | 7.02 | 169hr. |
| Example31 | Compound(1-146) | 3.60 | 764 | 7.64 | 6.66 | 165hr. |
| Example32 | Compound(1-147) | 3.68 | 802 | 8.02 | 6.86 | 158hr. |
| Example33 | Compound(1-148) | 3.65 | 765 | 7.66 | 6.60 | 153hr. |
| Example34 | Compound(1-149) | 3.62 | 786 | 7.86 | 6.81 | 164hr. |
| Example35 | Compound(1-150) | 3.64 | 813 | 8.12 | 7.01 | 167hr. |
| Example36 | Compound(1-151) | 3.62 | 804 | 8.04 | 6.98 | 170hr. |
| Example37 | Compound(1-152) | 3.67 | 749 | 7.49 | 6.41 | 158hr. |
| Example38 | Compound(1-153) | 3.61 | 758 | 7.58 | 6.60 | 167hr. |
| Comparative Example1 | Alq₃ | 3.65 | 714 | 7.14 | 6.15 | 114hr. |
| Comparative Example2 | Comparative Compound(2-1) | 3.67 | 731 | 7.30 | 6.25 | 135hr. |

As shown in Table 3, the driving voltage at a current density of 10 mA/cm² was almost the same for the devices in Comparative Examples 1 and 2, and Examples 21 to 38 using the heterocyclic compounds of the general formula (1) of the present invention as the capping layers. However, the luminance, the luminous efficiency, the power efficiency, and the device lifetime were significantly improved in the devices of Examples 21 to 38, as compared to the devices of Comparative Examples 1 and 2. This indicates that the heterocyclic compound of the general formula (1) of the present invention is a material which is suitably used for the capping layer, and with a high refractive index of the capping layer, therefore the light extraction efficiency of the organic EL devices can be significantly improved.

### Industrial Applicability

The heterocyclic compound represented by the general formula (1) has a high refractive index, which can significantly improve the light extraction efficiency and the stability in thin film state, and therefore is excellent as a compound suitably used in a capping layer of an organic EL device. With the organic EL device fabricated by using the heterocyclic compound represented by the general formula (1) of the present invention, a high efficiency can be obtained. The use of the heterocyclic compound represented by the general formula (1) of the present invention, which has no absorption in the blue, green, and red wavelength regions, makes it particularly suitable for displaying clear, bright images with good color purity. For example, it is now possible to develop applications in home appliances and lighting.

### Reference Sign List

- 1: Glass substrate
- 2: Metal Anode
- 3: Hole injection layer
- 4: Hole transport layer
- 5: Light emitting layer
- 6: Electron transport layer
- 7: Electron injection layer
- 8: Cathode
- 9: Capping layer

## Claims

1. A heterocyclic compound represented by the following general formula (1): 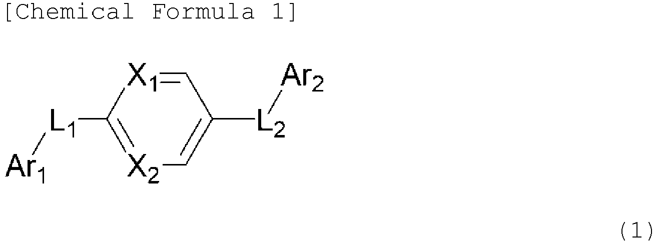 wherein X₁ and X₂ may be the same or different, and represent a nitrogen atom or a CH group, at least one of X₁ and X₂ is a nitrogen atom; L₁ and L₂ may be the same or different, and represent a single bond, or a divalent group that results from the removal of two hydrogen atoms from substituted or unsubstituted benzene, a divalent group that results from the removal of two hydrogen atoms from substituted or unsubstituted biphenyl, or a divalent group that results from the removal of two hydrogen atoms from substituted or unsubstituted naphthalene; Ar₁ and Ar₂ may be the same or different, and represent a substituted or unsubstituted aromatic hydrocarbon group selected from the group consisting of a naphthyl group, a phenanthrenyl group, and a spirobifluorenyl group, or a substituted or unsubstituted aromatic heterocyclic group selected from the group consisting of a quinolyl group, a benzofuranyl group, a benzothienyl group, a carbazolyl group, a dibenzothienyl group, a phenanthrolinyl group, a benzoxazolyl group, and a benzothiazolyl group.

2. The heterocyclic compound according to claim 1, wherein the heterocyclic compound is represented by the following general formula (1-a): 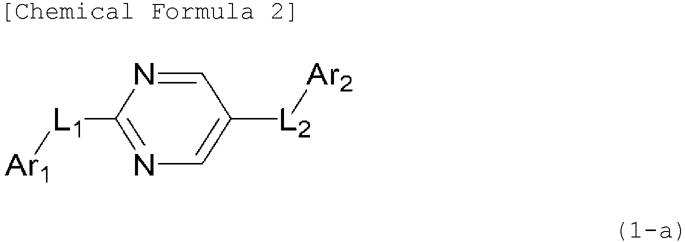 wherein L₁, L₂, Ar₁ and Ar₂ are as defined in the general formula (1).

3. The heterocyclic compound according to claim 1, wherein the heterocyclic compound is represented by the following general formula (1-b): 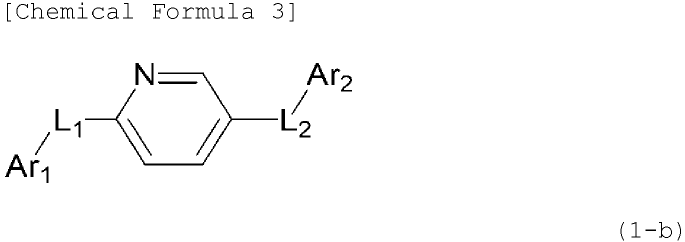 wherein L₁, L₂, Ar₁ and Ar₂ are as defined in the general formula (1).

4. The heterocyclic compound according to any one of claims 1 to 3, wherein in the general formula (1), in the general formula (1-a), or in the general formula (1-b), at least one of L₁ and L₂ is represented by the following the general formula (2): 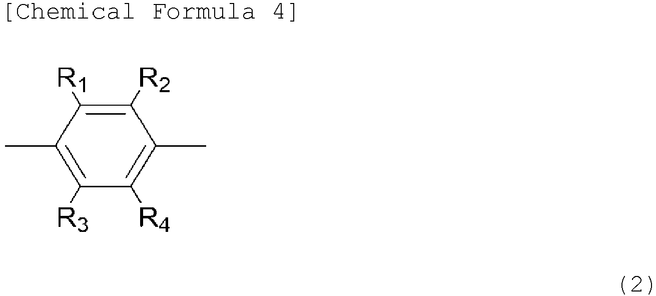 wherein R₁ to R₄ represent a hydrogen atom, a cyano group, a substituted or unsubstituted alkyl group of 1 to 6 carbon atoms, a substituted or unsubstituted alkyloxy group of 1 to 6 carbon atoms, or a substituted or unsubstituted aromatic hydrocarbon group of 6 to 12 ring-forming carbon atoms.

5. An organic thin film comprising the heterocyclic compound according to any one of claims 1 to 4, wherein the organic thin film has a refractive index of 1.70 or more at a wavelength in range of 450 nm to 750 nm.

6. An organic electroluminescent device comprising at least an anode electrode, a hole transport layer, a light emitting layer, an electron transport layer, a cathode electrode, and a capping layer in this order, wherein the capping layer is the organic thin film according to claim 5.

7. An electronic apparatus or an electronic device comprising a pair of electrodes and at least one organic layer sandwiched therebetween, wherein the heterocyclic compound according to any one of claims 1 to 4 is used for the organic layer as a constituent material thereof.

## Patentansprüche

1. Heterocyclische Verbindung, dargestellt durch die folgende allgemeine Formel (1): 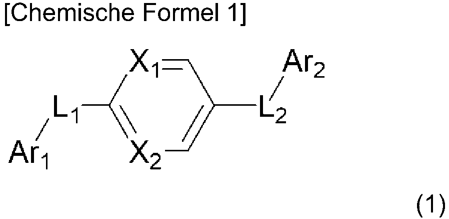 wobei X₁ und X₂ gleich oder verschieden sein können und ein Stickstoffatom oder eine CH-Gruppe darstellen, wobei mindestens eines von X₁ und X₂ ein Stickstoffatom ist; L₁ und L₂ gleich oder verschieden sein können und eine Einfachbindung oder eine bivalente Gruppe, die aus der Entfernung von zwei Wasserstoffatomen aus substituiertem oder unsubstituiertem Benzol resultiert, eine bivalente Gruppe, die aus der Entfernung von zwei Wasserstoffatomen aus substituiertem oder unsubstituiertem Biphenyl resultiert, oder eine bivalente Gruppe, die aus der Entfernung von zwei Wasserstoffatomen aus substituiertem oder unsubstituiertem Naphthalin resultiert, darstellen; Ar₁ und Ar₂ gleich oder verschieden sein können und eine substituierte oder unsubstituierte aromatische Kohlenwasserstoffgruppe, ausgewählt aus der Gruppe, bestehend aus einer Naphthylgruppe, einer Phenanthrenylgruppe und einer Spirobifluorenylgruppe, oder eine substituierte oder unsubstituierte aromatische heterocyclische Gruppe, ausgewählt aus der Gruppe, bestehend aus einer Quinolylgruppe, einer Benzofuranylgruppe, einer Benzothienylgruppe, einer Carbazolylgruppe, einer Dibenzothienylgruppe, einer Phenanthrolinylgruppe, einer Benzoxazolylgruppe und einer Benzothiazolylgruppe, darstellen.

2. Heterocyclische Verbindung nach Anspruch 1, wobei die heterocyclische Verbindung durch die folgende allgemeine Formel (1-a) dargestellt ist: 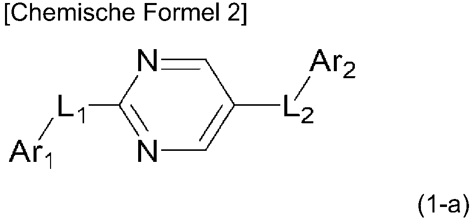 wobei L₁, L₂, Ar₁ und Ar₂ wie in der allgemeinen Formel (1) definiert sind.

3. Heterocyclische Verbindung nach Anspruch 1, wobei die heterocyclische Verbindung durch die folgende allgemeine Formel (1-b) dargestellt ist: 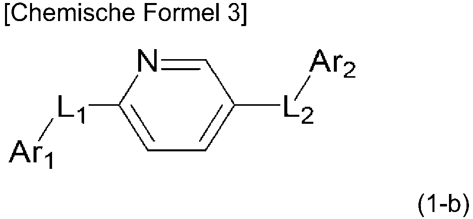 wobei L₁, L₂, Ar₁ und Ar₂ wie in der allgemeinen Formel (1) definiert sind.

4. Heterocyclische Verbindung nach einem der Ansprüche 1 bis 3, wobei in der allgemeinen Formel (1), in der allgemeinen Formel (1-a) oder in der allgemeinen Formel (1-b) mindestens eines von L₁ und L₂ durch die folgende allgemeine Formel (2) dargestellt ist: 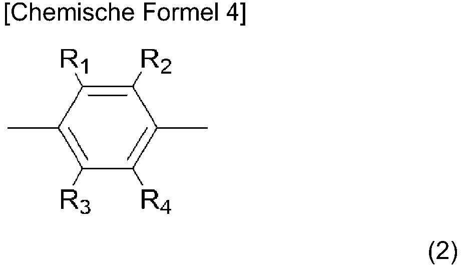 wobei R₁ bis R₄ ein Wasserstoffatom, eine Cyanogruppe, eine substituierte oder unsubstituierte Alkylgruppe mit 1 bis 6 Kohlenstoffatomen, eine substituierte oder unsubstituierte Alkyloxygruppe mit 1 bis 6 Kohlenstoffatomen oder eine substituierte oder unsubstituierte aromatische Kohlenwasserstoffgruppe mit 6 bis 12 ringbildenden Kohlenstoffatomen darstellen.

5. Organischer Dünnfilm, umfassend die heterocyclische Verbindung nach einem der Ansprüche 1 bis 4, wobei der organische Dünnfilm einen Brechungsindex von 1,70 oder mehr bei einer Wellenlänge im Bereich von 450 nm bis 750 nm aufweist.

6. Organische Elektrolumineszenzvorrichtung, umfassend mindestens eine Anodenelektrode, eine Lochtransportschicht, eine lichtemittierende Schicht, eine Elektronentransportschicht, eine Kathodenelektrode und eine Deckschicht in dieser Reihenfolge, wobei die Deckschicht der organische Dünnfilm nach Anspruch 5 ist.

7. Elektronischer Apparat oder elektronische Vorrichtung, umfassend ein Paar von Elektroden und mindestens eine dazwischen angeordnete organische Schicht, wobei die heterocyclische Verbindung nach einem der Ansprüche 1 bis 4 für die organische Schicht als ein konstituierendes Material davon verwendet wird.

## Revendications

1. Composé hétérocyclique représenté par la formule générale (1) suivante : 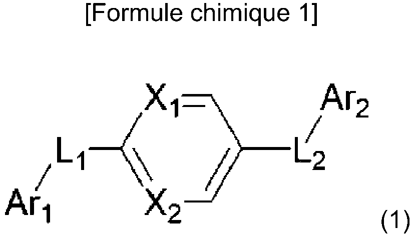 dans lequel X₁ et X₂ peuvent être identiques ou différents, et représentent un atome d'azote ou un groupe CH, au moins l'un de X₁ et X₂ est un atome d'azote ; L₁ et L₂ peuvent être identiques ou différents, et représentent une liaison simple, ou un groupe divalent qui résulte de l'élimination de deux atomes d'hydrogène d'un benzène substitué ou non substitué, un groupe divalent qui résulte de l'élimination de deux atomes d'hydrogène d'un biphényle substitué ou non substitué, ou un groupe divalent qui résulte de l'élimination de deux atomes d'hydrogène d'un naphtalène substitué ou non substitué ; Ar₁ et Ar₂ peuvent être identiques ou différents, et représentent un groupe hydrocarboné aromatique substitué ou non substitué choisi dans le groupe constitué par un groupe naphtyle, un groupe phénanthrényle et un groupe spirobifluorényle, ou un groupe hétérocyclique aromatique substitué ou non substitué choisi dans le groupe constitué par un groupe quinolyle, un groupe benzofuranyle, un groupe benzothiényle, un groupe carbazolyle, un groupe dibenzothiényle, un groupe phénanthrolinyle, un groupe benzoxazolyle et un groupe benzothiazolyle.

2. Composé hétérocyclique selon la revendication 1, dans lequel le composé hétérocyclique est représenté par la formule générale (1-a) suivante : 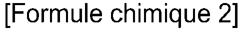 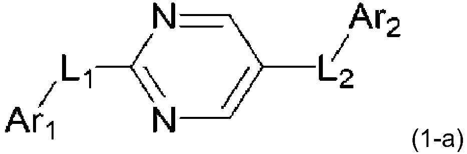 dans lequel L₁, L₂, Ar₁ et Ar₂ sont tels que définis dans la formule générale (1).

3. Composé hétérocyclique selon la revendication 1, dans lequel le composé hétérocyclique est représenté par la formule générale (1-b) suivante : 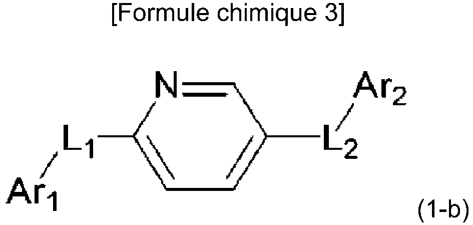 dans lequel L₁, L₂, Ar₁ et Ar₂ sont tels que définis dans la formule générale (1).

4. Composé hétérocyclique selon l'une quelconque des revendications 1 à 3, dans lequel dans la formule générale (1), dans la formule générale (1-a), ou dans la formule générale (1-b), au moins l'un de L₁ et L₂ est représenté par la formule générale (2) suivante : 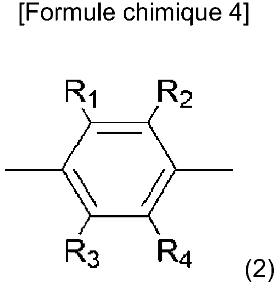 dans lequel R₁ à R₄ représentent un atome d'hydrogène, un groupe cyano, un groupe alkyle substitué ou non substitué de 1 à 6 atomes de carbone, un groupe alkyloxy substitué ou non substitué de 1 à 6 atomes de carbone, ou un groupe hydrocarboné aromatique substitué ou non substitué de 6 à 12 atomes de carbone formant cycle.

5. Film mince organique comprenant le composé hétérocyclique selon l'une quelconque des revendications 1 à 4, dans lequel le film mince organique présente un indice de réfraction de 1,70 ou plus à une longueur d'onde dans la plage de 450 nm à 750 nm.

6. Dispositif électroluminescent organique comprenant au moins une électrode d'anode, une couche de transport de trous, une couche émettrice de lumière, une couche de transport d'électrons, une électrode de cathode et une couche de recouvrement dans cet ordre, dans lequel la couche de recouvrement est le film mince organique selon la revendication 5.

7. Appareil électronique ou un dispositif électronique comprenant une paire d'électrodes et au moins une couche organique intercalée entre celles-ci, dans lequel le composé hétérocyclique selon l'une quelconque des revendications 1 à 4 est utilisé pour la couche organique en tant que matériau constitutif de celle-ci.
